# EUROPÄISCHE PATENTSCHRIFT

(11) **EP 1 308 166 B1**
(45) Veröffentlichungstag und Bekanntmachung des Hinweises auf die Patenterteilung: **26.08.2009**
(21) Anmeldenummer: 01130006.8
(22) Anmeldetag: 17.12.2001
(51) Int. Cl.: A61K 38/49, C12N 9/72, A61P 9/00, A61P 9/10

(54) **Nicht-neurotoxische Plasminogen Aktivierende Faktoren zur Behandlung von Schlaganfällen**
Non-neurotoxic plasminogen activating factors for treating stroke
Facteurs non neurotoxiques activateurs du plasminogène pour le traitement de l'accident vasculaire cérébral

(30) Priorität: 02.11.2001 DE 10153601
(43) Veröffentlichungstag der Anmeldung: 07.05.2003
(73) Patentinhaber: Paion Deutschland GmbH, 52062 Aachen (DE)
(72) Erfinder: Schleuning, Wolf-Dieter, DE-13467 Berlin (DE); Medcalf, Robert, Mentone 3194 Victoria (AU); Söhngen, Mariola, D-52080 Aachen (DE); Söhngen, Wolfgang, D-52080 Aachen (DE)
(74) Vertreter: König, Gregor Sebastian

(56) Entgegenhaltungen:
- EP-A- 0 273 774
- EP-A- 0 352 119
- EP-A- 0 383 417
- EP-A- 0 386 240
- DE-A- 3 917 949
- DE-A- 4 123 845
- US-A- 5 676 947
- US-B1- 6 235 279
- MEDEN P ET AL: "THROMBOLYSIS WITH RECOMBINANT DESMODUS SALIVA PLASMINOGEN ACTIVATOR (RDSPAALPHA1) IN A RAT EMBOLIC STROKE MODEL" CEREBROVASCULAR DISEASES, KARGER, BASEL,, CH, Bd. 6, 1996, Seite 190,AB67 XP008011404 ISSN: 1015-9770
- KE S H ET AL: "Identification of a hydrophobic exosite on tissue type plasminogen activator that modulates specificity for plasminogen." THE JOURNAL OF BIOLOGICAL CHEMISTRY. UNITED STATES 17 JAN 1997, Bd. 272, Nr. 3, 17. Januar 1997 (1997-01-17), Seiten 1811-1816, XP002232775 ISSN: 0021-9258
- STRANDBERG L ET AL: "Variants of tissue-type plasminogen activator with substantially enhanced response and selectivity toward fibrin co-factors." THE JOURNAL OF BIOLOGICAL CHEMISTRY. UNITED STATES 6 OCT 1995, Bd. 270, Nr. 40, 6. Oktober 1995 (1995-10-06), Seiten 23444-23449, XP002232776 ISSN: 0021-9258
- TACHIAS K ET AL: "VARIANTS OF TISSUE-TYPE PLASMINOGEN ACTIVATOR WHICH DISPLAY SUBSTANTIALLY ENHANCED STIMULATION BY FIBRIN" JOURNAL OF BIOLOGICAL CHEMISTRY, AMERICAN SOCIETY OF BIOLOGICAL CHEMISTS, BALTIMORE, MD, US, Bd. 270, Nr. 31, 4. August 1995 (1995-08-04), Seiten 18319-18322, XP002063362 ISSN: 0021-9258
- SCHLEUNING W-D: "VAMPIRE BAT PLASMINOGEN ACTIVATOR DSPA-ALPHA-1 (DESMOTEPLASE): A THROMBOLYTIC DRUG OPTIMIZED BY NATURAL SELECTION" HAEMOSTASIS, BASEL, CH, Bd. 31, Nr. 3-6, 2001, Seiten 118-122, XP008012096
- MADISON E L ET AL: "CONVERTING TISSUE PLASMINOGEN ACTIVATOR TO A ZYMOGEN: A REGULATORY TRIAD OF ASP-HIS-SER" SCIENCE, AMERICAN ASSOCIATION FOR THE ADVANCEMENT OF SCIENCE,, US, Bd. 262, 15. Oktober 1993 (1993-10-15), Seiten 419-421, XP000615414 ISSN: 0036-8075
- TOSCHI L ET AL: "Fibrin selectivity of the isolated protease domains of tissue-type and vampire bat salivary gland plasminogen activators." EUROPEAN JOURNAL OF BIOCHEMISTRY / FEBS. GERMANY 15 FEB 1998, Bd. 252, Nr. 1, 15. Februar 1998 (1998-02-15), Seiten 108-112, XP002232777 ISSN: 0014-2956
- THOMSON A: "NEW THROMBOLYTIC DRUGS" AUSTRALIAN AND NEW ZEALAND JOURNAL OF MEDICINE, ROYAL AUSTRALASIAN COLLEGE OF PHYSICIANS, SYDNEY, AU, Bd. 29, Nr. 3, 1999, Seiten 433-435, XP001010483 ISSN: 0004-8291

## Beschreibung

Die Erfindung betrifft eine therapeutische Anwendung nicht-neurotoxischer Plasminogen-Aktivatoren, insbesondere aus dem Speichel von *Desmodus rotundus* (DSPA), zur Behandlung von Schlaganfall.

Unter dem Begriff "Schlaganfall" werden verschiedene Krankheitsbilder zusammengefaßt, die sich in ihrer klinischen Symptomatik ähneln. Eine erste Differenzierung dieser Krankheitsbilder in die sogenannten ischämischen Insulte und hämorrhagischen Insulte ist ausgehend von der jeweiligen Pathogenese möglich.

Bei ischämischen Insulten (Ischämie) handelt es sich um eine Verminderung oder Unterbrechung der Durchblutung des Gehirns infolge mangelnder arterieller Blutzufuhr. Diese wird häufig durch eine Thrombose eines arteriosklerotisch stenosierten Gefäßes, aber auch durch arterioarterielle bzw. kardiale Embolien verursacht.

Die hämorrhagischen Insulte gehen dagegen u.a. auf eine Perforation der durch arterielle Hypertonie beschädigten, Hirn versorgenden Arterien zurück. Von allen zerebralen Insulten werden aber lediglich etwa 20% durch diese Form der Blutungen verursacht, so daß den durch Thrombose verursachten Schlaganfällen die weitaus größte Bedeutung zukommt.

Die Ischämie neuronaler Gewebe geht - im Vergleich zu anderen Gewebe-Ischämien - in besonderem Umfang mit der Nekrose der betroffenen Zellen einher. Die erhöhte Nekroseinzidenz kann nach neuerem Verständnis durch das Phänomen der sog. Exzitotoxizität erklärt werden, bei der es sich um eine komplexe Kaskade einer Vielzahl von Reaktionsschritten handelt. Diese wird demnach dadurch ausgelöst, daß die unter Sauerstoffmangel leidenden ischämischen Neuronen schnell ATP verlieren und depolarisieren. Dies führt zu einer verstärkten postsynaptischen Abgabe des Neurotransmitters Glutamat, der seinerseits membrangebundene Glutamat-Rezeptoren aktiviert, die Kationenkanäle regulieren. Als Folge der erhöhten Glutamat-Ausschüttung werden die Glutamat-Rezeptoren jedoch *über*aktiviert.

Die Glutamat-Rezeptoren regulieren ihrerseits spannungsabhängige Kationenkanäle, die - bei der Bindung von Glutamat an seinen Rezeptor - geöffnet werden. Damit setzt ein Na⁺ und ein Ca²⁺-Einstrom in die Zelle ein, der zu einer massiven Störung des Ca²⁺-abhängigen zellulären Stoffwechsels - einschließlich des Energiestoffwechsels - führt. Für den nachfolgend eintretenden Zelltod könnte dabei insbesondere die Aktivierung Ca²⁺-abhängiger katabolischer Enzyme verantwortlich sein (Lee, Jin-Mo et al., "The changing landscape of ischemic brain injury mechanisms"; Dennis W. Zhol "Glutamat neurotoxicity and deseases of the nervous system").

Selbst wenn der Mechanismus der Glutamat vermittelten Neurotoxizität derzeit noch nicht im Detail verstanden wird, scheint dennoch Einigkeit darüber zu bestehen, daß dieses Phänomen in einem erheblichen Umfang zu dem neuronalen Zelltod nach einer zerebralen Ischämie beiträgt (Jin-Mo Lee et. al.).

Bei der Therapie der akuten zerebralen Ischämie steht neben der Sicherstellung vitaler Funktionen und der Stabilisierung physiologischer Parameter zunächst das Bestreben nach einer Wiedereröffnung des verschlossenen Gefäßes im Vordergrund. Dieses Ziel wird mit verschiedenen Ansätzen verfolgt. Die rein mechanische Wiederveröffentlichung hat bisher noch keine zufriedenstellenden Ergebnisse erzielt, wie etwa die PTCA bei Herzinfarkt. Lediglich mit erfolgreicher Fibrinolyse konnte bisher eine ausreichende Besserung des Zustands für die Patienten aufgezeigt werden. Dies kann durch lokale Applikation mittels Katheter geschehen (PROCAT, eine Studie mit Pro-Urokinase), dieses Verfahren hat bisher jedoch trotz erster positiver Ergebnisse nicht zur Zulassung einer pharmazeutischen Substanz geführt.

Die körpereigene Fibrinolyse basiert auf der proteolytischen Aktivität der SerinProtease Plasmin, die durch eine Katalyse (Aktivierung) aus der inaktiven Vorstufe Plasminogen hervorgeht. Die natürliche Aktivierung des Plasminogens erfolgt durch die körpereigenen Plasminogen-Aktivatoren u-PA (Urokinase-typ Plaminogenaktivator) und t-PA (*Tissue Plasminogen Aktivator*). Letzterer bildet - im Gegensatz zu u-PA - gemeinsam mit Fibrin und Plasminogen einen sog. Aktivatorkomplex. Die katalytische Aktivität des t-PA ist demnach Fibrin-abhängig und erfährt in Gegenwart von Fibrin eine etwa 550-fache Steigerung. Neben Fibrin kann auch Fibrinogen die t-PA vermittelte Katalyse von Plasmin zu Plasminogen stimulieren - wenn auch in sehr viel geringerem Umfang. So erfährt die t-PA Aktivität in Gegenwart von Fibrinogen lediglich eine 25-fache Steigerung. Die Spaltprodukte des Fibrins (*fibrin degradation products* (FDP)) stimulieren ihrerseits ebenfalls die t-PA Aktivität.

Frühe Ansätze der thrombolytischen Behandlung des akuten Schlaganfalls gehen bereits auf die 1950-iger Jahre zurück. Aber erst ab 1995 wurden erste umfangreiche klinische Versuche mit Streptokinase, einem Fibrinolytikum aus beta-hämolysierenden Streptokokken, durchgeführt. Streptokinase bildet mit Plasminogen einen Komplex, der in der Lage ist, andere Plasminogenmoleküle in Plasmin umzusetzen.

Die Therapie mit Streptokinase ist aber mit wesentlichen Nachteilen verbunden, da Streptokinase als bakterielle Protease im Körper allergische Reaktionen auslösen kann. Auch kann eine sog. Streptokinase-Resistenz bei vorheriger Streptokokken-Infektion mit entsprechender Antikörperbildung vorliegen, die eine Therapie erschweren. Darüber hinaus deuteten klinische Studien in Europa (*Multicenter Acute Stroke Trial of Europe* (MAST-E), *Multicenter Acute Stroke Trial of Italy* (MAST-I)) und Australien (*Australien Streptokinase Trial* (AST)) auf ein derart erhöhtes Mortalitätsrisiko und die erhebliche Gefahr von intrazerebralen Blutungen (*intracerebral hemorrhage,* ICH) nach der Patienten-Behandlung mit Streptokinase hin, daß diese Studien sogar frühzeitig abgebrochen werden mußten.

In einem alternativen Therpieansatz wird Urokinase - ebenso ein "klassisches" Fibrinolytikum - appliziert, die im Gegensatz zu Streptokinase keine antigenen Eigenschaften aufweist, da es sich dabei um ein in zahlreichen Geweben vorkommendes körpereigenes Enzym handelt. Es stellt einen Kofaktor unabhängigen Aktivator des Plasminogens dar. Urokinase wird in Nierenzellkulturen hergestellt.

Umfangreiche Erfahrungen im Bereich der therapeutischen Thrombolyse liegen mit dem Gewebe-typ Plasminogen-Aktivator - dem sog. rt-PA - vor (siehe EP 0 093 619, US-Patentschrift 4 766 075), der in rekombinanten Hamsterzellen produziert wird. Mit t-PA wurden - neben der Hauptindikation akuter Myokardinfarkt - in den 1990-iger Jahren weltweit eine Reihe klinischer Studien mit zum Teil noch unverstandenen und widersprüchlichen Ergebnissen durchgeführt. So wurden zunächst im sog. *European Acute Stroke Trial* (ECASS) Patienten innerhalb eines Zeitraums von sechs Stunden nach dem Einsetzen der Symptome des Schlaganfalls intravenös mit rt-PA behandelt und nach 90 Tagen die Mortalitätsrate sowie der Barthel-Index als Maßstab für die Versehrtheit oder selbständige Lebensfähigkeit der Patienten untersucht. Dabei ergab sich keine signifikante Verbesserung der Lebensfähigkeit, sehr wohl aber eine - wenn auch nicht signifikante - Erhöhung der Mortalität. Dies erlaubte die Schlußfolgerung, daß eine thrombolytische Behandlung von individuell nach ihrer jeweiligen Krankengeschichte gezielt ausgesuchten Patienten mit rt-PA unmittelbar nach dem Beginn des Schlaganfalls möglicherweise vorteilhaft sein könnte. Aufgrund des beobachteten erhöhten Risikos einer intrazerebralen Haemorrhagie (ICH) nach nur wenigen Stunden nach Einsetzen des Schlaganfalls war aber von einer *generellen* Anwendung von rt-PA innerhalb des untersuchten Zeitraums von sechs Stunden nach Beginn des Schlaganfalls abzuraten (so C. Lewandowski C und Wiliam Barsan, 2001: Treatment of Acute Strocke; in: Annals of Emergency Medcine 37:2; S. 202 ff).

Die thrombolytische Behandlung des Schlaganfalls war später ebenso Gegenstand einer klinischen Studie des *National Institute of Neurologic Disorder and Stroke* (sog. *NINDS rtPA Stroke Trial*) in den USA. Dabei stand jedoch die Untersuchung der Wirkung einer intravenösen rt-PA-Behandlung innerhalb eines nur dreistündigen Zeitraums nach Beginn der Symptome auf den Gesundheitszustand der Patienten nach drei Monaten im Vordergrund. Obwohl die Autoren auch hier ein erhöhtes Risiko für ICH feststellten, wird dennoch die rt-PA Behandlung innerhalb dieses begrenzten Zeitraums von drei Stunden aufgrund der ebenfalls durch die Studie erkannten positiven Effekte dieser Behandlung auf die Lebensfähigkeit der Patienten empfohlen.

In zwei weiteren Studien (ECASS II Trial; *Alteplase Thrombolysis for Acute Noninterventional Therapy in Ischemic Stroke* (ATLANTIS)) wurde untersucht, ob sich diese positive Aussage über die Wirkung der rt-PA Behandlung innerhalb von drei Stunden nach Beginn des Schlaganfalls auch für ein Behandlung im sechsstündigen Zeitraum bestätigen ließen. Diese Frage konnte jedoch nicht positiv beantwortet werden, da durch eine Behandlung in diesem Zeitraum neben dem erhöhten Risiko für ICH keine Verbesserung der klinischen Symptome oder eine Verringerung der Mortalität beobachtet werden konnte.

Diese z.T. widersprüchlichen Ergebnisse haben zu einer großen Vorsicht in der Anwendung des rt-PA geführt. So wurde bereits 1996 in einer Publikation der *American Heart Association* konstatiert, daß unter den Ärzten hinsichtlich der thrombolytischen Behandlung des Schlaganfalls eine ausgeprägte Skepsis herrsche, die im Bezug auf die Therapie des Myokardinfarkts mit Fibrinolytika nicht bestehe (van Gijn J, MD, FRCP, 1996- Circulation 1996, 93: 1616-1617).

Eine Begründung dieser Skepsis findet sich in einer erstmalig 1997 publizierten und im März 2001 aktualisierten Zusammenschau aller "Stroke Trials". Danach führten alle Thrombolytika-Behandlungen (Urokinase, Streptokinase, rt-PA oder rekombinate Urokinase) insbesondere aufgrund von ICH zu einer signifikant erhöhten Mortalität innerhalb der ersten zehn Tage nach dem Schlaganfall, während sich bei einer Behandlung innerhalb des 6-Stunden-Zeitraums die Gesamtanzahl der entweder toten oder behinderten Patienten reduzierte. Von einer breiten Anwendung von Thrombolytika für eine Schlaganfallbehandlung wird daher abgeraten.

Diese Ergebnisse haben schon vorher andere Autoren zu der eher sarkastischen Aussage bewogen, daß Schlaganfall-Patienten nunmehr die Wahl hätten, zu sterben oder behindert zu überleben (SCRIP 1997: 2265, 26).

Gleichwohl stellt die Therapie mit rt-PA derzeit die einzige von der *Food and Drug Administration* (FDA) in den USA zugelassene Behandlungsmethode der akuten zerebralen Ischämie dar. Diese ist allerdings beschränkt auf eine Applikation des rt-PA innerhalb von drei Stunden nach dem Beginn des Schlaganfalls.

Die Zulassung des rt-PA erfolgte 1996. Unmittelbar zuvor, nämlich im Jahre 1995, wurden erste Hinweise auf schädliche Nebenwirkungen des t-PA bekannt, die einen Erklärungsansatz für die dramatischen Effekte des t-PA bei der Schlaganfallbehandlung außerhalb des 3-Stunden Behandlungszeitraums liefern. Danach produzieren Mikrogliazellen und neuronale Zellen des Hippocampus körpereigenes t-PA, das an der Glutamat vermittelten Exzitotoxizität beteiligt ist. Diese Schlußfolgerung entstand durch einen Vergleich von t-PA defizienten Mäusen und Wildtypmäusen, denen jeweils Glutamat-Agonisten in den Hippocampus injiziert wurden. Dabei zeigten die t-PA defizienten Mäuse eine deutlich erhöhte Resistenz gegen extern (inthrathekal) appliziertes Glutamat (Tsirka SE et. al., Nature, Vol. 377, 1995, "Exzitoxin-induced neuronal degeneration and seizure are mediated by tissue plasminogen activator"). Diese Ergebnisse wurden im Jahre 1998 bestätigt, als Wang *et al.* bei t-PA defizienten Mäusen durch die intravenöse Injektion von t-PA nahezu eine Verdoppelung des nekrotischen neuronalen Gewebes nachweisen konnten. Dieser negative Effekt des externen t-PA lag bei Wildmäusen dagegen nur etwa bei 33% (Wang et al., 1998, Nature, "Tissue plasminogen activator (t-PA) increases neuronal damage after focal cerebral ischemia in wild type and t-PA deficient mice").

Weiterführende Ergebnisse über die Stimulation der Exzitotoxizität durch t-PA publizierten Nicole *et al*. zu Beginn des Jahres 2001 (Nicole O; Docagne F Ali C; Margaill I; Carmeliet P; MacKenzie ET; Vivien D und Buisson A, 2001: The proteolytic activity of tissue-plasminogen activator enhances NMDA receptor-mediated signaling; in: Nat Med 7, 59-64). Sie konnten nachweisen, daß von depolarisierten kortikalen Neuronen ausgeschüttetes t-PA mit der sogenannten NR1 Untereinheit des Glutamat Rezeptors des NMDA Typs interagiert und diese spaltet. Diese Modifikation führt zu einer Erhöhung der Rezeptoraktivität, die ihrerseits für eine erhöhte Gewebeschädigung nach der Applikation des Glutamat Agonisten NMDA durch eine induzierte Exzitotoxizität verantwortlich ist. Das t-PA wirkt somit durch die Aktivierung des Glutamat-Rezeptor des NMDA-Typs neurotoxisch.

Nach einem weiteren Erklärungsansatz ist die Neurotoxizität des t-PA mittelbar auf die Aktivierung des Plasmins aus dem Plasminogen zurückzuführen. Eigentlicher Effektor der Neurotoxizität ist nach diesem Modell demnach das Plasmin (Chen ZL und Strickland S, 1997: Neuronal Death in the hippocampus is promoted by plasmin-catalysed degradation of laminin. Cell:91, 917-925).

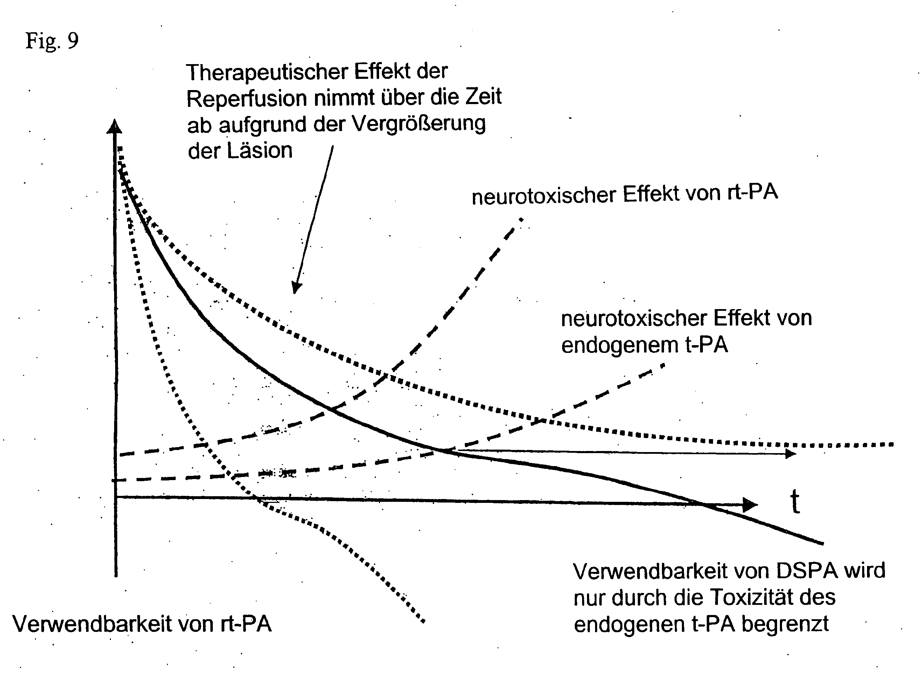

Eine zusammenfassende Darstellung der zeitabhängigen neurotoxischen Effekte des t-PA zeigt die Abb. 9. Darin wird zudem die erhöhte Toxizität des rekombinanten t-PA im Vergleich zum endogenen t-PA deutlich, die vermutlich darauf zurückzuführen ist, daß rt-PA in höheren Konzentrationen in das Gewebe eintritt.

Dass trotz dieser Hinweise auf die neurotoxischen Nebeneffekte des t-PA und trotz der nachgewiesenen Erhöhung der Mortalität durch t-PA dennoch eine arzneimittelrechtliche Zulassung durch die FDA erfolgte, kann wohl nur durch den Mangel an ungefährlichen und effektiven Alternativen - und eine sehr pragmatische Kosten-Nutzen Analyse - erklärt werden. Es besteht aber nach wie vor Bedarf für sicherere Therapien, wobei bei der Entwicklung neuer Thrombolytika - sofern denn von der Thrombolyse nicht sogar ganz Abstand zu nehmen ist - das Problem der Neurotoxizität berücksichtigt werden muß (so z.B. Wang et al a.a.O.; Lewandowski und Barsan 2001 a.a.O).

Aus diesem Grund unterblieben weiterführende Untersuchungen bekannter Thrombolytika einschließlich des DSPA (*Desmodus rotundus* Plasminogen Aktivator) zur Entwicklung eines neuen Therapeutikums für Schlaganfall, obwohl prinzipiell sämtliche Thrombolytika geeignet sein könnten und beispielsweise im Falle des DSPA bereits in einer ersten Veröffentlichung auf dessen mögliche Eignung für dieses Indikationsgebiet hingewiesen wurde (Medan P; Tatlisumak T; Takano K; Carano RAD; Hadley SJ; Fisher M: Thrombolysis with recombinant Desmodus saliva plasminogen activator (rDSPA) in a rat embolic stroke model; in: Cerebrovasc Dis 1996:6; 175-194 (4^{th} International Symposium on Thrombolic Therapy in Acute Ishemic Stroke)). Gerade bei DSPA handelt es sich aber um einen Plasminogen Aktivator mit hoher Homologie (Ähnlichkeit) zu t-PA, so daß - zugleich mit der Ernüchterung über die neurotoxischen Nebenwirkungen des t-PA - auch in DSPA keine weiteren Hoffnungen gesetzt wurden.

Vielmehr wird in jüngster Zeit u.a. die Strategie verfolgt, zur Verbesserung der bisherigen thrombolytischen Schlaganfallbehandlung mit den bekannten Thrombolytika die Substanzen nicht mehr intravenös, sondern intra-arteriell über einen Katheter direkt an den intravaskulären Thrombus zu bringen. Erste Erfahrungen dazu liegen mit der rekombinant erzeugten Urokinase vor. Damit kann möglicherweise die für die Thrombolyse erforderliche Dosis gesenkt und damit ein Teil der Nebenwirkungen reduziert werden. Diese Applikationsform erfordert jedoch einen hohen technischen Aufwand und steht daher nicht überall zur Verfügung. Zudem erfordert sie eine gewisse Zeit für die eingehende Vorbereitung des Patienten, die nicht immer zur Verfügung steht und stellt ein zusätzliches Risiko dar.

Ferner sind derzeitige Bestrebungen auf Antikoagulantien wie Heparin, Aspirin oder Ancrod, dem Wirkstoff aus dem Gift der Malayischen Grubenotter, ausgerichtet. Zwei u.a. auch auf die Untersuchung der Wirkungen von Heparin ausgerichtete klinische Studien (*International Stroke Trial* (IST) und *Trial of ORG 10172 in Acute Stroke Treatment* (TOAST)) deuten jedoch nicht auf eine signifikante Verbesserung der Mortalität und eine Verhinderung eines erneuten Schlaganfalls hin.

Eine weitere neuartige Behandlungsmethode setzt weder an dem Thrombus noch an der Blutverflüssigung oder Antikoagulation an, sondern versucht, die Vitalität der durch die Unterbrechung der Blutzufuhr geschädigten Zellen zu steigern (WO 01/51613 A1 und WO 01/51614 A1). Dazu werden Antibiotika aus der Gruppe der Quinone, Aminoglycoside oder Chloramphenicol appliziert. Aus einem ähnlichen Grund wird zudem vorgeschlagen, unmittelbar nach dem Schlaganfall mit der Gabe von Citicholin zu beginnen, das im Körper zu Cytidin und Cholin gespalten wird. Diese Spaltprodukte sind Bestandteile der neuronalen Zellmembran und können so die Regeneration des geschädigten Gewebes unterstützen (US-Patentschrift 5 827 832).

Jüngste Bestrebungen auf der Suche nach sicheren Behandlungsmethoden basieren auf den neuen Erkenntnissen darüber, daß ein Teil der fatalen Folgen des Schlaganfalls nur mittelbar auf die unterbrochene Blutzufuhr unmittelbar aber auf die Exzito- oder Neurotoxizität unter Beteiligung des überangeregten Glutamat-Rezeptors zurückzuführen ist, die wiederum insbesondere auch durch t-PA verstärkt wird (s. o). Ein Ansatz zur Verminderung dieser Exzitotoxizität ist daher die Applikation von sog. Neuroprotektiva. Diese können allein oder in Kombination mit Fibrinolytika verwendet werden, um so deren neurotoxischen Effekt zu minimieren. Sie können dabei entweder direkt, beispielsweise als Glutamat-Rezeptor-Antagonisten oder indirekt über die Blockade von spannungsabhängigen Natrium- oder Kalziumkanälen zu einer Abschwächung der Exzitotoxizität führen (Jin-Mo Lee et. al. a.a.O).

Eine kompetitive Hemmung (Antagonisierung) des Glutamat-Rezeptors des NMDA Typs ist dabei beispielsweise mit 2-amino-5-Phosphonovalerat (APV) oder 2-amino-5-Phosphonoheptanoate (APH) möglich. Eine nicht-kompetitive Hemmung kann beispielsweise durch Substanzen erreicht werden, die an die Phencyclidin Seite der Kanäle binden, wie Phencyclidin, MK-801, Dextrorphan oder Ketamin.

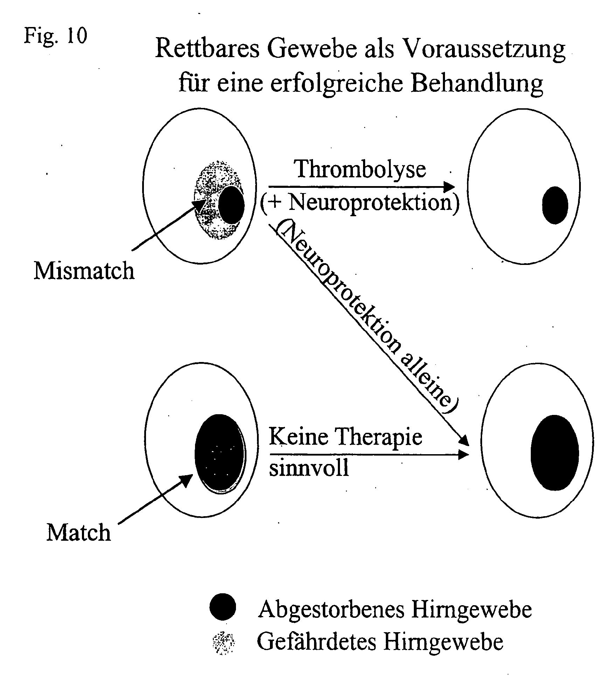

Bislang haben die Behandlungen mit Neuroprotektiva jedoch noch nicht den gewünschten Erfolg erbracht, möglicherweise weil sie mit Thrombolytika kombiniert werden müssten, um ihre protektive Wirkung zu entfalten. Dies gilt im gleichen Maße für die anderen Wirkstoffe auch (siehe Fig. 10).

Selbst mit einer Kombination aus t-PA und einem Neuroprotektivum ermöglicht jedoch allenfalls nur einen Erfolg im Sinne einer Schadensbegrenzung. Die Nachteile der Neurotoxizität des verwendeten Fibrinolytikums selber werden damit aber nicht vermieden.

Der vorliegenden Erfindung liegt daher die Aufgabe zugrunde, einen neuen therapeutischen Ansatz zur Behandlung des Schlaganfalls beim Menschen bereitzustellen.

Diese Aufgabe wird gelöst durch die Verwendung von Mutanten eines Plasminogen Aktivierenden Faktor gemäß dem Hautanspruch zur therapeutischen Behandlung von Schlaganfall. Weitere vorteilhafte Verwendungen sind jeweils Gegenstand weiterführender Unteransprüche.

Kern der Erfindung ist es, einen Plasminogenaktivator nach Ablauf von 3 Stunden seit Beginn des Schlaganfalls für die Schlaganfallbehandlung einzusetzen, dessen Fibrinspezifität im Vergleich zum Wildtyp t-PA erhöht ist, ohne daß ein weiteres Therapeutikum verwendet wird,

Die erfindungsgemäße Verwendung dieses Plasminogen-Aktivators basiert auf der Erkenntnis, daß die Neurotoxizität des GewebePlasminogen-Aktivators (t-PA) darauf zurückzuführen ist, daß durch die aufgrund des Schlaganfalls verursachte Gewebezerstörung im Gehirn die Blut-Hirn-Schranke beschädigt oder zerstört wird und das im Blut zirkulierende Fibrinogen somit in das neuronale Gewebe des Gehirns eindringen kann. Dort aktiviert es t-PA, das - mittelbar durch die Aktivierung des Glutamat-Rezeptors oder durch die Aktivierung des Plasminogens - zu weiteren Gewebeschädigungen führt. Zur Vermeidung dieses Effekts wird nun erfindungsgemäß ein Plasminogen-Aktivator eingesetzt, der eine erhöhte Fibrinselektivität und - im Umkehrschluß - eine verringerte Aktivierbarkeit durch Fibrinogen zeigt. Daraus folgt, daß der erfindungsemäße Plasminogen-Aktivator beim Übertritt von Fibrinogen aus dem Blut in das neuronale Gewebe in Folge der beschädigten Blut-Hirn-Schranke nicht - oder in einem im Vergleich zum t-PA deutlich verringertem Ausmaß - aktiviert wird, da sein Aktivator Fibrin aufgrund seiner Größe nicht in das neuronale Gewebe eintreten kann. Die erfindungsgemäßen Plasminogen-Aktivatoren sind somit nicht-neurotoxisch.

In einer bevorzugten Ausführungsform werden nicht-toxische Plasminogen-Aktivatoren eingesetzt, die wenigstens ein Element einer sog. Zymogen-Triade aufweisen. Eine vergleichbare Triade ist aus dem katalytischen Zentrum von Serinproteasen der Chymotrypsin-Familie bekannt, die aus den drei interagierenden Aminosäuren Aspartat 194, Histidin 40 und Serin 32 besteht. Diese Triade ist bei dem zu der Familie der Chymotrypsin-artigen Serinproteasen gehörenden t-PA jedoch nicht vorhanden. Es ist aber bekannt, daß die gezielte Mutagenese des nativen t-PA zur Einführung wenigstens einer dieser Aminosäuren an geeigneten Positionen zu einer Verringerung der Aktivität des Pro-Enzyms (einketttiges t-PA) und einer Erhöhung der Aktivität des reifen Enzyms (zweikettiges t-PA) bei Anwesenheit von Fibrin führt. Somit führt die Einführung wenigstens einer Aminosäure aus der Triade - oder von Aminosäuren, die die entsprechende Funktion in der Triade übernehmen - zu einer Erhöhung der Zymogenität des t-PA (Verhältnis der Aktivität des reifen Enzyms zu der Aktivität des Pro-Enzyms) bei deutlicher Erhöhung der Fibrinspezifität, durch konformative Wechselwirkung zwischen den eingeführten Aminosäureresten und/oder Aminosäureresten der wildtypischen Sequenz.

So ist es bekannt, daß die Mutagenese des nativen t-PA zur Substitution des Phe305 zu His (F305H) sowie des Ala 292 zu Ser (A292S) zu einer 20-fachen Erhöhung der Zymogenität führt, während aber bereits bei der Variante F305H allein eine Steigerung um das 5-fache erzeugt wird (EL Madison, Kobe A, Gething M-J, Sambrook JF, Goldsmith EJ 1993: Converting Tissue Plasminogen Activator to a Zymogen: A regulatory Triad of Asp-His-Ser; Science: 262, 419-421). Diese t-PA Mutanten zeigen bei der Anwesenheit von Fibrin eine Aktivitätssteigerung um das 30.000-fache (F305H) bzw. um das 130.000-fache (F305H, A292S). Beide Mutanten weisen zusätzlich eine Substitution des Arg275 zu R275E auf, um die Spaltung des einkettigen t-PA in die zweikettige Form an der Spaltungsstelle Aug275-Ile276 durch Plasmin zu verhindern. Allein diese Mutante R275E erhöht die Fibrinspezifität des t-PA um das 6.900-fache (K Tachias, Madison EL 1995: Variants of Tissue-type Plasminogen Activator Which Display Substantially Enhanced Stimulation by Fibrin, in: Journal of Biological Chemistry 270, 31: 18319-18322).

Die Positionen 305 und 292 des t-PA sind homolog zu den Positionen His40 und Ser32 der bekannten Triade der Serinproteasen. Durch die entsprechende Substitution mit Histidin bzw. Serin können diese Aminosäuren mit dem Aspartat477 des t-PA interagieren, so daß die bekannte Triade funktionell in den t-PA Mutanten ausgebildet werden kann (Madison et al 1993).

Erfindungsgemäß können diese t-PA-Mutanten zur Behandlung des Schlaganfalls eingesetzt werden, da sie aufgrund ihrer erhöhten Fibrinspezifität keine oder - im Vergleich zum Wildtyp t-PA - eine nur deutlich verringerte Neurotoxizität aufweisen. Zu Zwecken der Offenbarung der erwähnten t-PA Mutanten F305H; F305H, A292S allein oder in Kombination mit R275E werden die Veröffentlichungen von Madison et al 1993 sowie Tachias und Madison 1995 vollumfänglich in bezug genommen.

Die Erhöhung der Fibrinspezifität von Plasminogen-Aktivatoren ist alternativ ebenso möglich durch eine Punktmutation des Asp194 (oder eines Aspartats an homologer Position). Plasminogen-Aktivatoren gehören zu der Gruppe der Serinproteasen der Chymptrypsin-Familie und weisen übereinstimmend die konservierte Aminosäure Asp194 auf, die für die Stabilität der katalytisch aktiven Konformation der reifen Proteasen verantwortlich ist. Es ist bekannt, daß das Asp194 in den Zymogenen der Serinproteasen mit His40 interagiert. Durch die aktivierende Spaltung des Zymogens werden diese Wechselwirkungen unmöglich, und die Seitenkette des Asp194 um etwa 170° rotiert, um anschließend mit dem Ile16 eine neue Salzbrücke auszubilden. Diese Salzbrücke ist an der Stabilität der Oxyanion-Tasche der katalytischen Triade der reifen Serinprotease beteiligt. Sie liegt auch im t-PA vor.

Eine Punktmutation des Asp194 macht die Ausbildung bzw. Stabilität der katalytischen Konformation der Serinproteasen zunächst unmöglich. Gleichwohl zeigen die mutierten Plasminogen-Aktivatoren bei Anwesenheit ihres Co-Faktors Fibrin - gerade auch im Vergleich zu der reifen Wildtyp-Form - eine deutliche Aktivitätssteigerung, die nur damit zu erklären ist, daß die Wechselwirkungen mit Fibrin eine katalytische Aktivität ermöglichende Konformationsänderung erlaubt (L Strandberg, Madison EL, 1995: Variants of Tissue-type Plasminogen Activator with Substantially Enhanced Response and Selectivity towards Fibrin Co-factors. in: Journal of Biological Chemistry 270, 40: 2344-23449). Somit zeigen die Asp194-Mutanten der Plasminogen-Aktivatoren eine hohe Aktivitätsteigerung bei Anwesenheit von Fibrin, die ihre erfindungsgemäße Verwendung ermöglicht.

In einer bevorzugten Ausführungsform wird t-PA erfindungsgemäß eingesetzt, dessen Asp194 durch Glutamat (D194E) bzw. Asparagin (D194N) substituiert ist. Damit reduziert sich bei Abwesenheit von Fibrin die Aktivität von t-PA um den Faktor 1-2000, während dagegen bei Anwesenheit von Fibrin eine Aktivitätssteigerung um den Faktor 498.000 bis 1.050.000 erreicht werden kann. Diese Mutanten können zudem eine Substitution des Arg15 zu R15E enthalten, die die Spaltung des einkettigen t-PA zu dem zweikettigen t-PA an der Peptidbindung Arg15-Ile16 durch Plasmin verhindert. Durch diese Mutation allein wird die Aktivierung des t-PA durch Fibrin bereits um den Faktor 12.000 erhöht. Zu Zwecken der Offenbarung der Mutationen an den Positionen 194 sowie 15 des t-PA wird die Veröffentlichung von Strandberg und Madison 1995 vollumfänglich in Bezug genommen.

Eine Erhöhung der Fibrin-Abhängigkeit von Plasminogen-Aktivatoren kann zudem über die Einführung von Punktmutationen in die sog. "Autolyse-Schleife" erreicht werden. Dieser Aminosäure-Abschnitt ist aus Trypsin bekannt; er liegt aber als homologer Abschnitt auch in Serinproteasen vor und ist insbesondere gekennzeichnet durch drei hydrophobe Aminosäuren (Leu, Pro und Phe). Die Autolyse-Schleife in Plasminogen-Aktivatoren ist für die Interaktion mit Plasminogen verantwortlich. Punktmutationen in diesem Bereich können dazu führen, daß die Protein/Protein Wechselwirkungen zwischen Plasminogen und Plasmimogen-Aktivator funktionell nicht mehr ausgebildet werden können. Diese Mutationen sind funktionell jedoch nur bei Abwesenheit von Fibrin relevant. Bei Anwesenheit von Fibrin sind sie dagegen für eine Aktivitätssteigerung der Plasminogen-Aktivatoren verantwortlich (K Song-Hua, Tachias K, Lamba D, Bode W, madison EL, 1997: Identifikation of a Hydrophobic exocite on Tissue Type Plasminogen Activator That Modulates Specificity for Plasminogen. In: Journal of Biological Chemistry 272; 3, 1811-1816).

In einer bevorzugten Ausführungsform wird ein t-PA mit Punktmutationen in den Positionen 420-423 eingesetzt. Werden diese Reste durch gezielte Punktmutationen substituiert, steigert sich die Fibrin-Abhängigkeit des t-PA um einen Faktor von bis zu 61.000 (K Song-Hua et al.). Song-Hua et al. untersuchten die Punktmutationen L420A, L420E, S421G, S421E, P422A, P422G, P422E, F423A und F423E. Die Veröffentlichung wird hiermit vollumfänglich für die erfindungsgemäße Verwendung in bezug genommen.

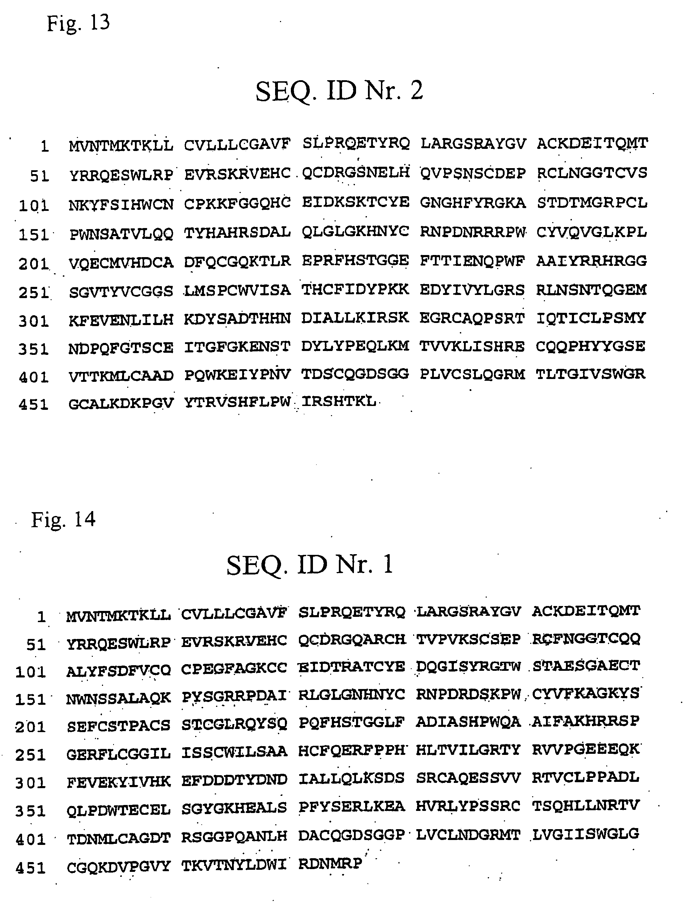

In einer weiteren vorteilhaften Ausführung wird modifizierter Gewebeplasminoginaktivator mit einer Aminosäuresequenz gemäß SEQ.ID Nr. 1 (Fig. 13) verwendet. Dieses modifizierte t-PA unterscheidet sich von dem Wildtyp-t-PA durch den Austausch der hydrophoben Aminosäure in den Positionen 420 - 423 in der Autolyse-Schleife, die wie folgt besetzt sind: His420, Asp421, Ala422 und Cys423. Dieses t-PA weist bevorzugt ein Phenylalanin an der Position 194 auf. Zudem kann die Position 275 durch ein Glutamat besetzt sein. Vorteilhafterweise ist die Position 194 von Phenylalanin besetzt.

Weiterhin kann eine modifizierte Urokinase erfindungsgemäß eingesetzt werden. Diese erfindungsgemäße Urokinase kann die Aminosäure gemäß SEQ. ID Nr. 2 (Fig. 14) aufweisen, in der die hydrophoben Aminosäuren der Autolyse-Schleife durch Val420, Thr421, Asp422 und Ser423 ersetzt sind. Vorteilhafterweise trägt diese Urokinase ein lle275 sowie ein Glu194. Diese Mutante zeigt im Vergleich zu der Wildtyp-Urokinase eine um das 500-fache erhöhte Fibrinspezifität.

Beide Mutanten - sowohl Urokinase als auch t-PA - wurden in semi-quantitativen Tests untersucht und ergaben eine im Vergleich zum Wildtyp t-PA erhöhte Fibrinspezifität.

Eine hohe Steigerung der Aktivität bei Anwesenheit von Fibrin - nämlich eine Steigerung um das 100.000-fache - zeigt zudem auch der Plasminogen-Aktivator (DSPA) aus dem Speichel der Vampirfledermaus (*Desmodus rotundus),* der daher bevorzugt erfindungsgemäß verwendet werden kann. Unter dem Begriff DSPA werden vier verschiedene Proteasen zusammengefaßt, die ein grundlegendes Bedürfnis der Vampirfledermaus, nämlich die verlängerte Blutungsdauer der von diesen Tieren verursachten Wunden der Beutetiere erfüllen (Cartwright, 1974). Diese vier Proteasen (DSPAα₁. DSPAα₂, DSPAß, DSPAγ) weisen übereinstimmend eine hohe Ähnlichkeit (Homologie) zu humanem t-PA auf. Ebenso weisen sie ähnliche oder übereinstimmende physiologische Aktivitäten auf, die ihre Zusammenfassung zu dem Oberbegriff DSPA rechtfertigen. DSPA ist Gegenstand der EP 0 352 119 A1 sowie der US Patente 6 008 019 und 5 830 849.

Bei DSPAα1 handelt es sich um die bisher am besten untersuchte Protease dieser Gruppe. Sie weist in ihrer Aminosäuresequenz eine Homologie von über 72% gegenüber der bekannten humanem t-PA Aminosäuresequenz auf (Krätzschmar et al., 1991). Dennoch bestehen zwischen t-PA und DSPA zwei wesentliche Unterschiede. Erstens nämlich stellt DSPA als Einzelkette gegenüber Peptidsubstraten ein voll aktives Molekül dar, das nicht - wie das t-PA - in eine zweikettige Form überführt wird (Gardell et al, 1989; Krätzschmar et al., 1991). Zweitens zeigt die katalytische Aktivität des DSPA eine nahezu absolute Fibrin Abhängigkeit (Gardell et al., 1989; Bringmann et al., 1995; Toschi et al., 1998). So wird beispielsweise die Aktivität des DSPAα1 bei Anwesenheit von Fibrin um das 100.000-fache gesteigert, während die t-PA-Aktivität nur um das etwa 550-fache ansteigt. Die DSPA-Aktivität wird dagegen durch Fibrinogen wesentlich weniger stark induziert; es erfährt nur eine Steigerung um das 7- bis 9-fache (Bringmann et al., 1995). DSPA ist somit erheblich stärker Fibrin abhängig und Fibrin spezifisch als das Wildtyp t-PA, das nur etwa 550-fach durch Fibrin aktiviert wird.

Aufgrund seiner fibrinolytischen Eigenschaften und der großen Ähnlichkeit zu t-PA stellt DSPA zwar einen interessanten Kandidaten für die Entwicklung eines Thrombolytikums dar. Gleichwohl wurde in der Vergangenheit die Entwicklung von DSPA als Thrombolytikum auf die Behandlung des Myokardinfarkts beschränkt, da - aufgrund der Beteiligung des t-PA an der Glutamat abhängigen Neurotoxizität - keine berechtigten Hoffnungen bestanden, einen mit t-PA verwandten Plasminogen Aktivator erfolgreich zur Behandlung des akuten Schlaganfalls einzusetzen.

Nun hat sich aber überraschenderweise gezeigt, daß DSPA trotz der hohen Ähnlichkeit (Homologie) zu t-PA und trotz der weitgehenden Übereinstimmung seiner physiologischen Wirkung zu t-PA im Gegensatz zu diesem keine neurotoxischen Wirkungen aufweist. Mit dieser Feststellung geht unmittelbar die Erkenntnis einher, daß DSPA als Thrombolytikum doch zur Therapie des Schlagfalls verwendet werden kann, ohne daß damit ein zusätzliches Risiko der Schädigung des neuronalen Gewebes verbunden wäre. Dies bedeutet insbesondere, daß DSPA auch später als drei Stunden nach dem Einsetzen der Symptome beim Schlaganfall eingesetzt werden kann.

Weitere Lehre der vorliegenden Erfindung ist die sich aus vorbeschriebenen Erkenntnis ergebende Möglichkeit auch andere Plasminogen Aktivatoren derart zu modifizieren oder herstellen, dass sie diese Eigenschaft des DSPA aufweisen bzw. die Neurotoxizität des tPA nicht aufweisen. Grundlage hierfür ist der erkannte Wirkzusammenhang, der es zukünftig erlaubt neurotoxische Plasminogen Aktivatoren in nicht-neurotoxische Plasminogen Aktivatoren umzuwandeln bzw. auf der Basis bekannter oder neu entdeckter neurotoxischer Plasminogen Aktivatoren nicht-neurotoxische Plasminogen Aktivatoren herzustellen.

Die neue Lehre basiert auf *in vivo*-Vergleichsuntersuchungen der neurodegenerativen Wirkung des t-PA einerseits und des DSPA andererseits, die unter Zuhilfenahme des sog. "Kainsäure-Modells" sowie eines Modells zur Untersuchung der NMDA induzierten Läsion des Striatums durchgeführt wurden.

Das Kainsäure-Modell (auch Kainsäure Verletzungsmodell) beruht darauf, daß die neurotoxische Glutamat Kaskade durch die Applikation von externer Kainsäure (KA) als Agonist des Glutamat-Rezeptors des Kainsäure Typs (KA-Typ) sowie der NMDA- und AMPA-Glutamat-Rezeptoren stimuliert wird. Durch die Verwendung eines t-PA defizienten Mäusestamms als Versuchsmodell konnte damit gezeigt werden, daß die Sensitivität der Versuchstiere gegen Kainsäure nur bei einer zusätzlichen Gabe externen t-PAs das Niveau der Wildtypmäuse erreichte. Dagegen konnte eine Infusion einer äquimolaren Konzentration an DSPA unter den gleichen experimentellen Bedingungen die Sensitivität gegenüber der Kain-Säure (KA) nicht wiederherstellen. Die Wirkung des t-PA konnten demnach durch DSPA nicht substituiert werden. Eine Zusammenfassung dieser Ergebnisse ist in Tabelle 2 dargestellt.

**Tabelle 2**

| | | **Hippocampal length intac (mm)** | | |
|---|---|---|---|---|
| **Treatment group** | **Number of animals** | **Contralateral side** mean (SEM) | **Ipsilateral sid mean (SEM)** | **Percentage remaining** |
| t-PA infusion (1.85 uM) + KA | 12 | 15.99 (0.208) | 3.63 (0.458) | 22.7* |
| DSPA infusion (1.85 uM) + KA | 11 | 16.07 (0.124) | 13.8 (0.579) | 85.87 |
| t-PA infusion (1.85 uM) + PBS | 3 | 16.75 (0.381) | 17.08 (0.363) | 101.97 |
| DSPA infusion (1.85 uM) + PBS | 3 | 15.75 (0.629) | 15.83 (0.363) | 100.50 |
| t-PA infusion (0.185 uM) + KA | 3 | 15.60 (0.702) | 5.07 (1.09) | 32.5 |
| DSPA infusion (18.5 uM) + KA | 3 | 16.06 (0.176) | 13.80 (1.22) | 85.93 |

| | | | | |
|---|---|---|---|---|
| * P < 0.0001 | | | | |

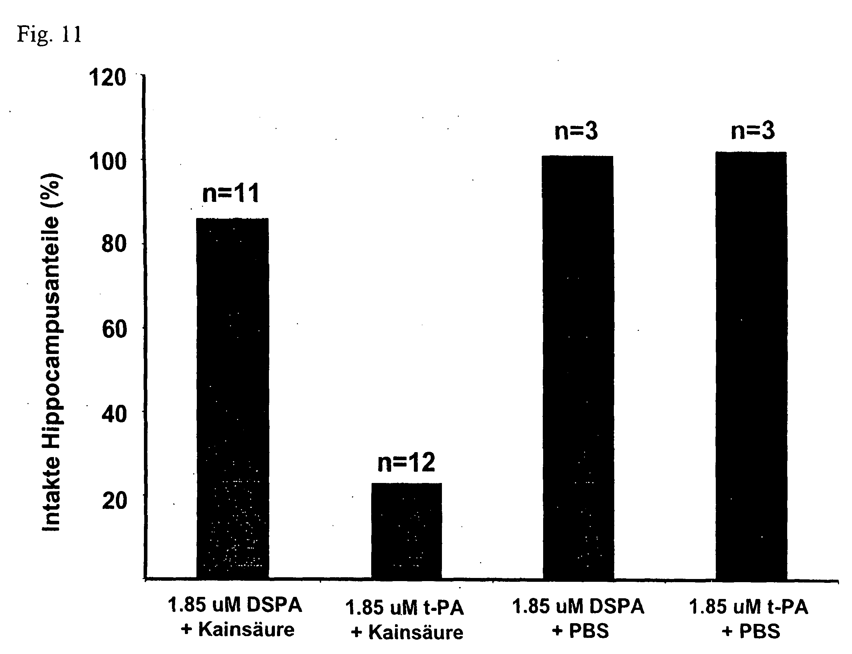

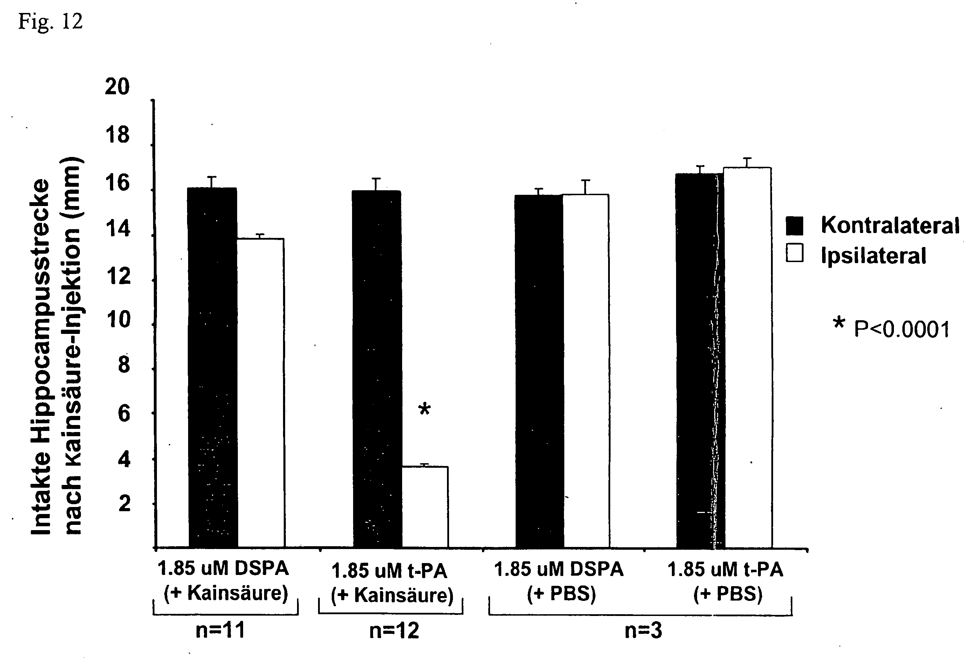

Quantitative Untersuchungen an diesem Modell zeigten, daß selbst eine 10-fache Steigerung der DSPA-Konzentration die Sensitivität der t-PA defizienten Mäusen gegen die KA-Behandlung nicht wiederherstellte, während aber bereits eine 10-fach niedrigere t-PA-Konzentration KA-induzierte Gewebeschäden herbeiführte. Daraus folgt, daß DSPA bei der Förderung der Neurodegeneration nach KA Behandlung eine wenigstens 100-fach niedrigere Aktivität als t-PA aufweist (siehe auch Figuren 11 und 12).

In dem zweiten Modell der Neurodegeneration wurden die möglichen Wirkungen des t-PA sowie des DSPA auf die Förderung der NMDA-abhängigen Neurodegeneration in Wildtypmäusen verglichen. Dazu wurde Wildtypmäusen NMDA (als Agonist des Glutamat Rezeptors des NMDA-Typs) allein oder in Kombination mit t-PA oder DSPA injiziert. Dieses Modell erlaubt die Untersuchung der Wirkung dieser Proteasen unter Bedingungen, unter denen eine Neurodegeneration ohnehin eintritt und aufgrund des Zusammenbruchs der Blut-Hirn-Schranke einen Einstrom von Plasmaproteinen verursacht (Chen et al., 1999).

Bei Arbeiten an diesem Modell führte die Injektion von NMDA zu reproduzierbaren Läsionen im Striatum der Mäuse. Das Volumen dieser Läsionen wurde durch eine gemeinsame Injektion von t-PA und NMDA um mindestens 50% vergrößert. Dagegen führte eine Co-Injektion mit DSPAα1 zu keiner Erhöhung der Vergrößerung der durch die NMDA-Injektion verursachten Läsionen. Sogar in Gegenwart von Plasma-Proteinen, die infolge der NMDA-abhängigen Neurodegeneration in das Läsionsgebiet diffundieren konnten, führte DSPA zu keinem Anstieg der Neurodegeneration (siehe auch Tabelle 3).

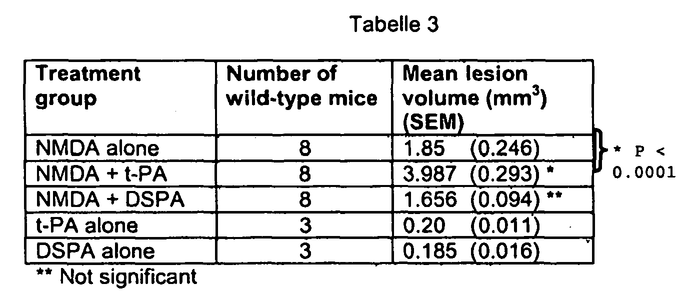

Diese Ergebnisse zeigen, daß DSPA im Zentralnervensystem eines Säugers - also auch des Menschen - eine im wesentlichen inerte Protease darstellt und - im Gegensatz zu t-PA - keine Erhöhung der von KA oder NMDA induzierten Neurotoxizität hervorruft. Diese fehlende Neurotoxizität macht DSPA entgegen der Erwartung zu einem geeigneten Thrombolytikum für die Behandlung des akuten Schlaganfalls.

Erste Ergebnisse aus klinischen Studien zeigen die Übertragbarkeit dieser Ergebnisse auch auf die Behandlung des Schlaganfalls beim Menschen. So sind bei Patienten nach erfolgreicher Perfusion deutliche Verbesserungen festzustellen (Verbesserung um 8 Punkte NIHSS oder NIHSS score 0 - 1). Dies macht die Tabelle 1 deutlich.

**Tabelle 1**

| Patient | | NIHSS | | | | |
|---|---|---|---|---|---|---|
| | Baseline | Post Tmt | Day 7 | Day 30 | Day 90 | sAEs |
| 1001 | 12 | 7 | 4 | 4 | * | Re-Infarction |
| 1002 | 8 | 9 | 2 | 0 | 0 | |
| 1003 | 8 | 10 | 12 | 10 | * | |
| 1004 | 8 | 4 | 2 | 0 | 0 | |
| 1005 | 11 | 11 | 4 | 5 | * | |
| 1006 | 9 | 7 | 1 | * | * | |
| 1007 | 14 | 6 | * | * | * | |
| 2001 | 19 | 20 | -- | -- | -- | ICH, death |
| 2002 | 15 | 21 | -- | -- | -- | ICH, death |
| 3001 | 8 | 7 | 6 | 5 | * | |
| 3002 | 15 | 16 | 9 | 8 | * | |
| 3003 | 10 | 19 | 21 | * | -- | Death day 39 |

Aufgrund der fehlenden Neurotoxizität des DSPA sowie der übrigen ebenso nicht-neurotoxischen Plasminogen-Aktivatoren (s. o.) ergibt sich als besonderer Vorteil für die Schlaganfallbehandlung die Tatsache, daß die Verwendung dieser Plasminogen-Aktivatoren - anders als die des Wildtyp t-PA - nicht nur auf das enge Zeitfenster von bis zu drei Stunden nach Beginn des Schlaganfalls beschränkt ist. Vielmehr kann die Behandlung auch zu einem späteren Zeitpunkt - so also ohne weiteres auch nach sechs Stunden oder noch später - erfolgen, ohne daß dem - wie bei t-PA - das Risiko einer Förderung der Exzitotoxizität entgegenstünde. Erste klinische Untersuchungen mit dem DSPA belegen sogar die unbedenkliche Behandlung von Patienten in einem Zeitraum von mehr als sechs bzw. neun Stunden nach dem Einsetzen der Symptome.

Diese zeitlich unbegrenzte Behandlungsoption mit nicht-neurotoxischen Plasminogen-Aktivatoren ist gerade deswegen so bedeutsam, da damit erstmals eine Behandlung von akuten Schlaganfallpatienten bedenkenlos möglich wird, bei denen der Beginn des Schlaganfalls zeitlich nicht mit ausreichender Sicherheit ermittelt werden kann. Diese Patientengruppe war bisher aus Gründen der Vorsicht und der Risikoabschätzung von der Thrombolyse mit Plasminogen-Aktivatoren ausgenommen worden. Damit entfällt eine wesentliche Kontraindikation für die zugelassene Verwendung eines Thrombolytikums bei Schlaganfall.

In einer weiteren vorteilhaften Ausführungsform können die nicht-neuorotoxischen Plasminogen-Aktivatoren mit einem pharmazeutisch unbedenklichen Träger kombiniert werden.

### VERGLEICHENDE UNTERSUCHUNGEN VON T-PA UND DSPA

### A. Methoden

### 1. Tiere

Wildtypmäuse (c57/Black 6) und t-PA defiziente Mäuse (t-PA-/- Mäuse) (c57/Black 6) (Carmeliet et al., 1994) wurden von Dr. Peter Carmeliet, Leuven, Belgien, zur Verfügung gestellt.

### 2. Protein Extraktion aus Gehirngewebe

Die Bestimmung der proteolytischen Aktivität im Hirngewebe nach Infusion von t-PA oder DSPAα1 erfolgte mittels zymographischer Analyse (Granelli-Piperno und Reich, 1974). Nach der 7-tägigen Infusionsdauer in den Hippocampus wurden die Mäuse betäubt, anschließend transcardial mit PBS perfundiert und die Gehirne entnommen. Die Hippocampus Region wurde entnommen, in Eppendorf Gefäße gegeben und in gleichen Volumina (w/v) (etwa 30-50 µl) mit 0.5% NP-40 Lyse Puffer ohne Protease-Inhibitoren (0.5% NP-40, 10 mM Tris-HCl pH 7.4, 10 mM NaCI, 3 mM MgCl₂, 1mM EDTA) inkubiert. Gehirn-Extrakte wurden mittels eines handbetriebenen Glass-Homogenisators homogenisiert und für 30 min auf Eis belassen. Die Proben wurden danach zentrifugiert und der Überstand abgenommen. Die vorliegende Protein-Menge wurde bestimmt (Bio-Rad Reagenz).

### 3. Zymographische Analyse der Proteasen

Die proteolytische Aktivität in den Proben oder den Extrakten des Gehirngewebes wurde durch zymographische Analyse gemäß dem Verfahren von Granelli-Pipemo und Reich (1974) bestimmt. Dabei wurden die Proben des rekombinanten Proteins (bis zu 100 nmol) oder des Gehimgewebe-Extrakts (20 µg) einer 10%igen SDS-PAGE unter nicht-reduzierenden Bedingungen unterzogen. Die Gele wurden aus den Platten entnommen, für zwei Stunden in 1% Triton X100 gewaschen und anschließend einem Agarosegel mit polymerisiertem Fibrinogen und Plasminogen aufgelegt (Granelli-Piperno und Reich, 1974). Die Gele wurden bei 37°C in einer Feuchtkammer inkubiert bis proteolysierte Zonen erkennbar wurden.

### 4. Intra-Hippocampus Infusion von t-PA, DSPA und anschließende Injektion von Kain-Säure

Das Kainsäure-Verletzungsmodell basiert auf dem Ansatz von Tsirka et al. (1995). Die Tiere wurden intraperitonal (i.p.) mit Atropin (4 mg/kg) injiziert, dann mit einer i.p. Injektion von Natrium Pentobarbital (70 mg/kg) betäubt. Anschließend wurden sie in einen stereotaktischen Rahmen eingesetzt, so daß eine mikroosmotische Pumpe (Alzet model 1007D, Alzet CA. USA) mit 100 µl PBS oder rekombinantem humanen t-PA (0.12 mg/ml; 1.85 µM) oder DSPAα1 (1.85 µM) subkutan zwischen die Schulterblätter implantiert werden konnte. Die Pumpen wurden durch sterile Röhrchen mit einer Gehirn-Kanüle verbunden und durch eine Schädelöffnung an den Koordinaten bregma -2.5 mm, midolateral 0.5 mm und dorsoventral 1.6 mm eingesetzt, um die Flüssigkeit nahe der Mittellinie einzuführen. Die Kanüle wurde nachfolgend an der gewünschten Position festgeklebt und die Pumpen geöffnet, um die entsprechenden Lösungen bei einer Flußrate von 0.5 µl pro Stunde über sieben Tage zu infundieren.

Zwei Tage nach der Infusion der Proteasen wurden die Mäuse erneut betäubt und in den stereotaktischen Rahmen eingebracht. 1.5 nmol Kainsäure in 0.3 µl PBS wurden anschließend unilateral in den Hippocampus injiziert. Die Koordinaten waren: bregma 2.5 mm, medial-lateral 1.7 mm und dorsoventral 1.6 mm. Das Exzitotoxin (KA) wurde über eine Dauer von 30 sec. zugeführt. Nach der Kainsäure-Behandlung verblieb die Nadel für weitere 2 min an diesen Koordinaten, um einen Rückfluß der Flüssigkeit zu verhindern.

### 5. Gehirn Untersuchung

Fünf Tage nach der KA Injektion wurde die Tiere betäubt und transkardial mit 30 ml PBS perfundiert, gefolgt von 70 ml einer 4% Paraformaldehydlösung, für 24 Stunden im gleichen Fixativ nachfixiert, gefolgt von einer Inkubation in 30% Sucrose für weitere 24 Stunden. Coronalschnitte (40 µm) des Gehirns wurden anschließend auf einem vereisbaren Microtom geschnitten, entweder mit Thionin (BDH, Australien) gegengefärbt oder für die immunohistochemische Untersuchung vorbereitet.

### 6. Quantifizierung der neuronalen Verlustrate innerhalb des Hippocampus

Die Quantifizierung des neuronalen Verlusts in den CA1-CA3 Hippocampus Subfeldern wurde wie zuvor beschrieben durchgeführt (Tsirka et al. 1995; Tsirka et al. 1996). Fünf aufeinander folgende Abschnitte des dorsalen Hippocampus aus allen behandelten Gruppen wurden vorbereitet, wobei die Abschnitte tatsächlich die Stelle der KA Injektion und das Gebiet der Läsion umfaßten. Die Hippocampus Subfelder (CA1-CA3) dieser Abschnitte wurden mittels Camera lucida Zeichnungen des Hippocampus verfolgt. Die Gesamtlänge dieser Subfelder wurde gemessen im Vergleich zu 1 mm Standards, die bei gleicher Vergrößerung verfolgt wurden. Die Länge der Gewebeabschnitte mit vitalen Pyramidenneuronen (mit normaler Morphologie) und die Länge der Gewebeabschnitte ohne Neuronen (keine Zellen anwesend, keine Thionin Färbung) wurde ermittelt. Die Längen, die intakte Neuronen und neuronale Verluste im Bereich jedes Hippocampus Subfeldes darstellten, wurden zwischen den Abschnitten gemittelt und die Standardabweichung bestimmt.

### 7. Intra-striatale NMDA Exzitoxizitäts-Läsionen mit oder ohne t-PA oder DSPA

Wildtypmäuse (c57/Black6) wurden betäubt und in einen stereotaktischen Rahmen eingesetzt (s.o.). Die Mäuse erhielten anschließend eine unilaterale Injektion in das linke Stratum mit 50 nmol NMDA allein oder in Kombination mit 46 µM rt-PA oder 46 µM DSPAα1. Als Kontrolle wurden zudem t-PA und DSPAα1 allein in einer Konzentration von 46 µM als Kontrollen injiziert. Die Injektions-Koordinaten waren: bregma -0.4 mm, midolateral 2.0 mm und dorsoventral 2.5 mm. Die Lösungen (1 µl Gesamtvolumen für alle Behandlungen) wurden über eine Zeitspanne von 5 Minuten bei 0.2 µl/min übertragen, wobei die Nadel weitere 2 min nach der Injektion am Injektionsort verblieb, um den Rückfluß von Flüssigkeit zu minimieren. Nach 24 Stunden wurden die Mäuse betäubt und transkardial mit 30 ml PBS perfundiert, gefolgt von 70 ml einer 4% Paraformaldehydlösung, einer Nach-Fixierung für 24 Stunden im gleichen Fixativ, gefolgt von einer Inkubation in 30% Sucrose für weitere 24 Stunden. Coronalschnitte (40 µm) wurden dann auf einem vereisbaren Microtom geschnitten und auf Gelatine beschichtete Glasträger aufgelegt.

### 8. Quantifizierung des Läsionsvolumens nach der Injektion von NMDA

Die Quantifizierung des striatalen Läsionsvolumens wurde anhand der von Callaway et al. (2000) beschriebenen Methode durchgeführt. Dabei wurden 10 aufeinander folgende Coronalabschnitte vorbereitet, die das Gebiet der Schädigung umfaßten. Die geschädigte Region wurde anhand der Callaway Methode visualisiert und das Läsionsvolumen durch Verwendung eines Micro Computer Imaging Device (MCID, Imaging Research Inc., Brock .University, Ontario, Canada) quantifiziert.

### 9. Immunhistochemie

Die Immunhistochemie wurde nach Standardmethoden durchgeführt. Coronalsektionen wurden einer Immersion in einer Lösung aus 3% H₂O₂/10% Methanol für 5 min unterzogen, gefolgt von einer Inkubation mit 5% normal Ziegenserum für 60 min. Die Schnitte wurden dann über Nacht entweder mit einem anti-GFAP Antikörper (1:1,000; Dako, Carpinteria, Ca, USA) zum Nachweis von Astrocyten, mit einem anti-MAC-1 Antikörper (1:1,000; Serotec, Raleigh, NC, USA) zum Nachweis von Mikroglia oder polyclonalen anti-DSPA Antikörpern (Schering AG, Berlin) inkubiert. Nach dem Waschen wurden die Schnitte mit den passenden biotinylierten sekundären Antikörpern (Vector Laboratories, Burlingame, CA, USA) inkubiert. Darauf folgte eine abschließende Inkubation in einem Avidin/Biotin-Komplex (Vector Laboratories, Burlingame, CA, USA) für 60 min vor der endgültigen Anfärbung mit 3,3'-Diaminobebzidin/0.03% H₂O₂. Die Schnitte wurden dann auf gelatine-beschichtete Träger übertragen, getrocknet, dehydriert und mit permount eingedeckelt.

### B. Ergebnisse

### 1. Infusion von t-PA oder DSPA wird über den Hippocampus von t-PA -/Mäusen verteilt und behält ihre proteolytische Aktivität

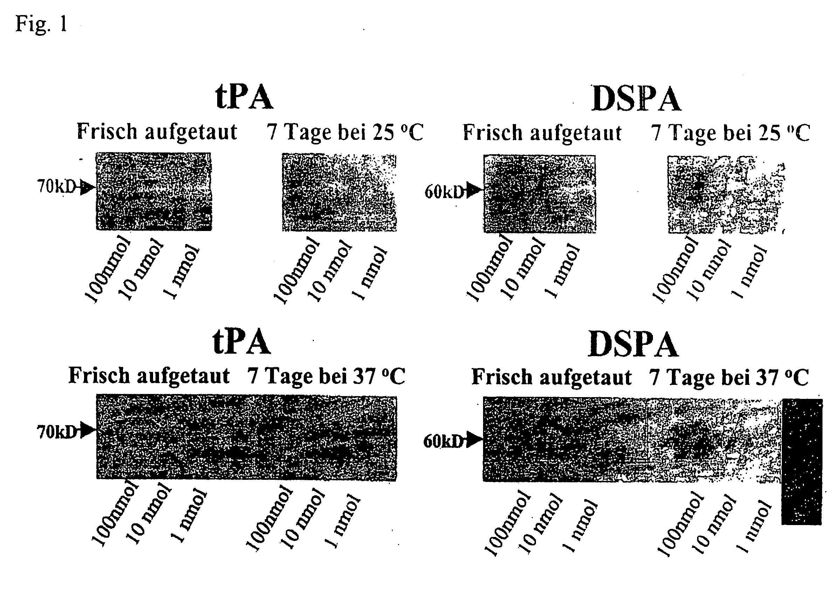

Die ersten Versuche wurden konzipiert, um zu bestätigen, daß sowohl DSPA als auch t-PA ihre proteolytische Aktivität über die 7-tägige Dauer der Infusion behalten. Zu diesem Zweck wurden Aliquots von t-PA und DSPA (100 nmol) sowohl bei 37°C als auch bei 30°C für sieben Tage in einem Wasserbad inkubiert. Um die proteolytische Aktivität zu bestimmen, wurden serielle 5-fache Verdünnungen der Proben einer SDS-PAGE mit nicht-reduzierenden Bedingungen unterworfen und die proteolytische Aktivität durch zymographische Analysen gemessen. Jeweils ein Aliquot von t-PA und DSPA, das während dieser sieben Tage eingefroren geblieben war, wurde als Kontrolle benutzt. Wie aus **Figur 1** ersichtlich ist, gab es nur einen geringen Verlust der DSPA- oder t-PA Aktivität, sowohl bei der Inkubation bei 25°C als auch bei 37°C über diesen Zeitraum.

### 2. t-PA und DSPA Aktivität ist auch in Hippocampus Extrakten von t-PA -/Mäusen nach der Infusion auffindbar

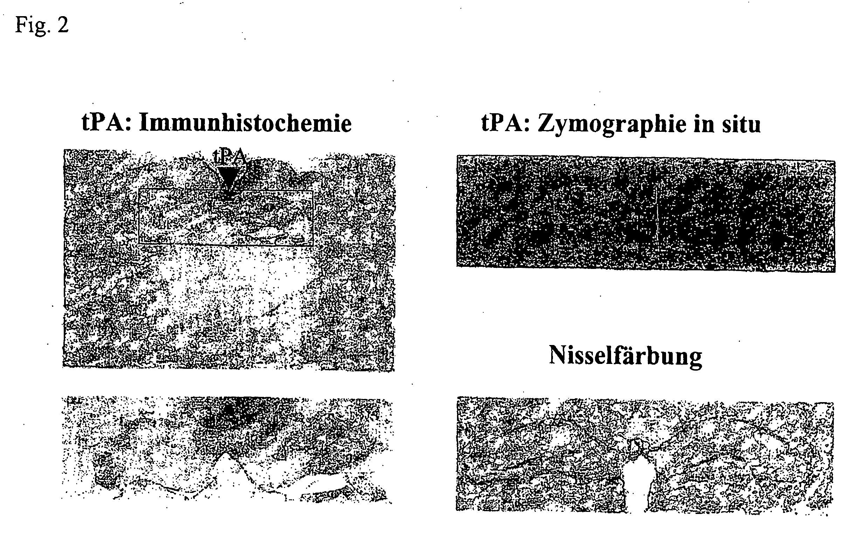

Zunächst mußte festgestellt werden, daß die bei der Infusion verabreichten Proteasen überhaupt im Gehirn der behandelten Tiere vorhanden waren und auch dort ihre proteolytische Aktivität behielten. Dazu erhielten t-PA -/- Mäuse sieben Tage lang Infusionen mit t-PA oder DSPA (s.o.). Die Mäuse wurden anschließend durch transkardiale Perfusion mit PBS getötet, und die Gehirne entnommen. Die ipsilateralen und contralateralen Hippocampus Regionen wurden isoliert, ebenso eine Region des Cerebellums (als Negativ-Kontrolle). Gewebeproben (20 µg) wurden einer SDS-PAGE und zymographischen Analyse gemäß der Beschreibung im Methodenteil unterworfen. Wie aus **Figur 2** ersichtlich, wurden sowohl t-PA als auch DSPA Aktivitäten im ipsilateralen Bereich des Hippocampus gemessen, wobei außerdem etwas Aktivität auf der contralateralen Seite gemessen wurde. Dies zeigte, daß die infundierten Proteasen nicht nur ihre Aktivität im Gehirn behalten, sondern sich außerdem in der Hippocampus Region ausgebreitet hatten. Bei der Kontrolle wurde keine Aktivität in dem aus dem Cerebellum präparierten Extrakt nachgewiesen.

### 3. Immunhistochemischer Nachweis von DSPA

Um nachzuweisen, daß DSPA sich tatsächlich über die Hippocampus Region ausgebreitet hatte, wurden coronale Gehirnschnitte von t-PA -/- Mäusen nach DSPA-Infusion immunohistochemisch analysiert. Dabei wurde DSPA Antigen in der Hippocampus Region nachgewiesen, wobei die Umgebung der Infusionsstelle die stärkste Färbung aufwies. Dieses Ergebnis bestätigt, daß das infundierte DSPA löslich ist und tatsächlich im Hippocampus vorliegt.

### 4. DSPA-Infusion stellt die Sensitivität gegenüber Kainsäure-abhängiger Neurodegeneration in vivo nicht wieder her

t-PA -/- Mäuse sind resistent gegenüber Kainsäure-abhängiger Neurodegeneration. Dagegen führt eine Infusion von rt-PA in den Hippocampus zu einer vollständigen Wiederherstellung der Sensitivität gegenüber Kainsäure-abhängiger Schädigung. Zur Beantwortung der Frage, ob DSPA das t-PA in dieser Wirkung ersetzen kann, erhielten t-PA -/- Mäuse mittels einer mini-osmotischen Pumpe Infusionen mit t-PA oder DSPA in den Hippocampus. Für beide Gruppen wurden 12 Mäuse verwendet. Zwei Tage später erhielten die Tiere Injektionen von Kainsäure bei einer sich anschließenden Ruhephase. Fünf Tage danach wurden die Tiere getötet. Die Gehirne wurden entnommen und aufgearbeitet (s.o.). Als Kontrollen wurden außerdem t-PA -/- Mäuse vor der KA Behandlung mit PBS infundiert (n=3).

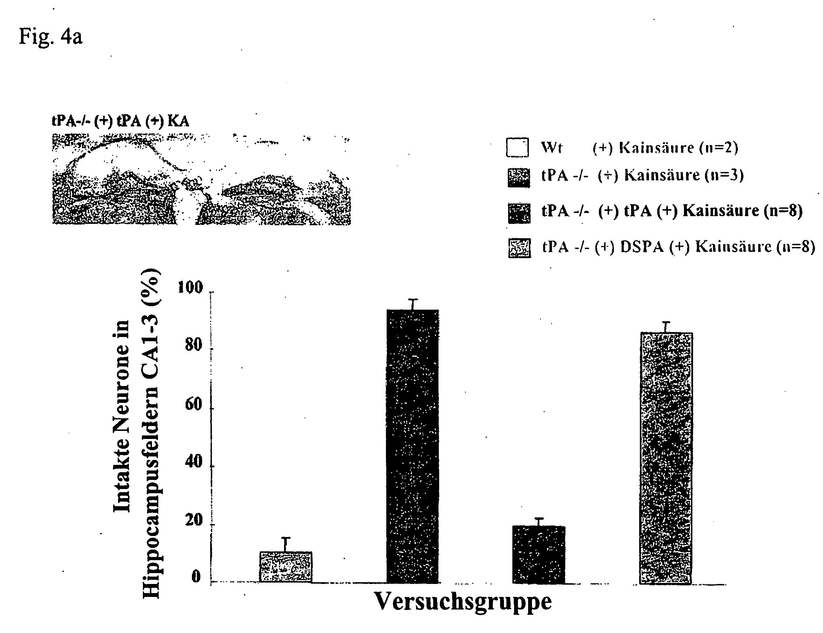

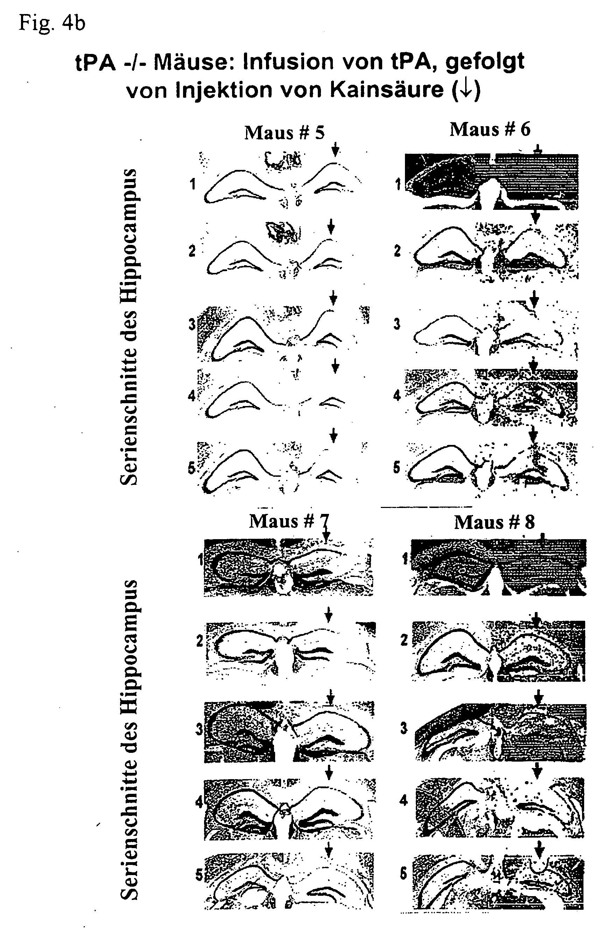

Es wurden coronale Gehirnschnitte präpariert und die Neuronen durch die Färbung nach Nissl detektiert. Es wurde deutlich, daß die t-PA -/- Mäuse nach Infusion von PBS resistent gegenüber KA waren. Dagegen führten Infusionen mit rekombinantem t-PA zur Wiederherstellung der Sensitivität gegenüber der Behandlung mit KA. Im Gegensatz dazu veränderte die Infusion der gleichen Konzentration an DSPA in die Hippocampus-Region die Sensitivität dieser Tiere gegenüber KA nicht (siehe Fig. 4a und. 4b).

Eine Quantifizierung dieser Ergebnisse erfolgte unter Verwendung von 12 Mäusen in jeder Gruppe. Bei 2 der 12 mit DSPA infundierten Mäusen beobachteten wir einige geringfügige Neurodegenerationen. Der Grund hierfür ist noch unklar und möglicherweise unabhängig von der Gegenwart von DSPA. Die kombinierten Daten berücksichtigen den geringfügigen Effekt, der bei diesen beiden Tieren beobachtet wurde. Alle 12 mit t-PA behandelten Mäuse waren empfindlich gegenüber der KA Behandlung. Diese Ergebnisse zeigen, daß bei einer Infusion von t-PA oder DSPAα1 in äquimolaren Konzentrationen nur die Gabe von t-PA zu einer Wiederherstellung der Sensitivität gegenüber KA-induzierter Neurodegeneration führt.

### 5. DSPA Infusion verursacht keine Mikroglia Aktivierung

Die Wiederherstellung der KA Sensitivität von t-PA -/- Mäusen nach t-PA Infusion resultiert nachweislich in einer Mikroglia-Aktivierung (Rogove et al., 1999). Um das Ausmaß der Mikroglia-Aktivierung nach t-PA- oder DSPA-Infusion und anschließender KA Behandlung zu bestimmen, wurden Coronal-Schnitte der Mäuse einer immunohistochemischen Färbung für aktivierte Microglia-Zellen unter Verwendung des Mac-1 Antikörpers unterworfen. Die Wiederherstellung der KA Sensitivität nach t-PA Infusion resultierte in einem klaren Anstieg der Mac-1 positiven Zellen. Dies wurde nicht bei den Mäusen beobachtet, die Infusionen mit DSPA erhalten hatten. Somit führt die Gegenwart von DSPA nicht zu einer Aktivierung von Mikroglia-Zellen nach KA Behandlung.

### 6. Titration von DSPA und t-PA in der Mäuse Hippocampus Region

Die Konzentration von t-PA, die für die Infusion benutzt wurde, basierte auf der von Tsirka et al (1995) beschriebenen Konzentration (100 µl von 0.12 mg/ml [1.85 µM]. Wir wiederholten die KA Belastungs-Experimente unter Verwendung einer 10-fach niedrigeren t-PA-Menge (0.185 µM) und einer höheren Menge an DSPA (18.5 µM). Die niedrigere t-PA-Konzentration war noch immer in der Lage, die Sensitivität gegenüber der KA-Behandlung wiederherzustellen (n=3). Von besonderem Interesse war, daß die Infusion der 10fach erhöhten DSPA-Konzentration nur geringen neuronalen Verlust nach KA Behandlung verursachte. Diese Daten weisen verstärkt darauf hin, daß DSPA die Sensitivität gegenüber KA nicht erhöht.

### 7. Auswirkung von t-PA und DSPA auf die NMDA-abhängige Neurodegeneration in Wildtypmäusen

Die Wirkungen von t-PA und DSPA wurden des weiteren in einem Modell der Neurodegeneration an Wildtypmäusen untersucht. Die Injektion von t-PA in das Striatum dieser Mäuse führt nachweislich zu einer Vertärkung der von dem Glutamat Analogen NMDA hervorgerufenen neurodegenerativen Effekte (Nicole et al., 2001).

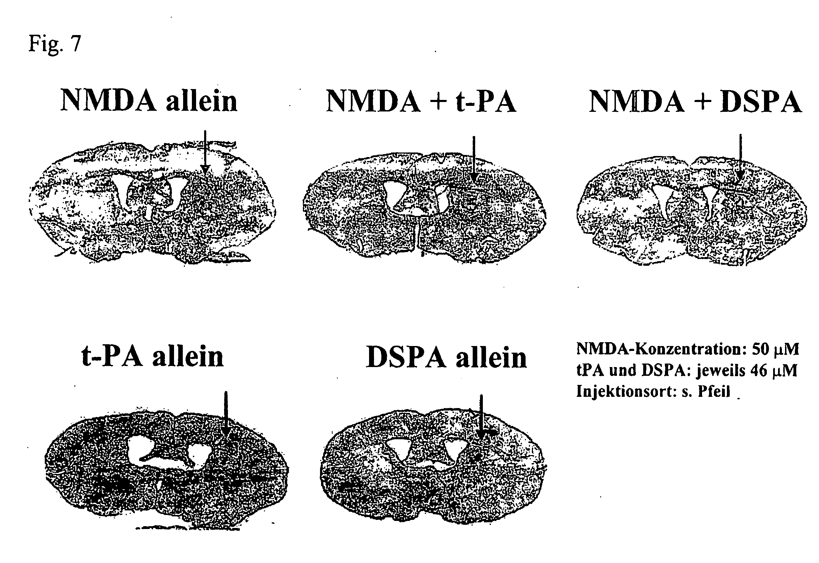

Dazu wurden NMDA-Injektionen mit einem Gesamtvolumen von 1 µl in die Striatum Region von Wildtypmäusen in Gegenwart von t-PA oder DSPA (jeweils 46 µM) verabreicht. Nach 24 Stunden wurden die Gehirne entnommen und die Größe der Läsionen nach der Callaway Methode (Callaway et al., 2000) quantifiziert (s.o.). Wie aus **Figur 7** ersichtlich, verursachte die Injektion von NMDA allein eine reproduzierbare Läsion bei allen behandelten Mäusen (n=4). Wurden t-PA und NMDA jedoch gemeinsam injiziert, nahm die Größe der Läsionen um etwa 50% zu (P<0.01, n=4). In deutlichem Gegensatz dazu verursachte die Co-Injektion von NMDA und der gleichen Konzentration von DSPA keinen Anstieg der Läsionsgröße im Vergleich zu NMDA allein.

Injektionen von t-PA oder DSPA allein führten nicht zu einer nachweisbaren Neurodegeneration. Die fehlende Wirkung von t-PA bei alleiniger Verabreichung ist konsistent mit den Ergebnissen von Nicole et al. (2001). Diese Daten zeigen, daß die Anwesenheit von DSPA die Neurodegeneration auch während eines neurodegenerativen Vorgangs nicht weiter erhöht.

Um zu bestätigen, daß sich die Injektion von DSPA tatsächlich in der Hippocampus Region ausgebreitet hat, wurden immunohistochemische Untersuchungen an Coronar-Schnitten unter Verwendung des DSPA Antikörpers durchgeführt. Untersuchungen zeigen, daß DSPA tatsächlich in die striatale Region eingedrungen ist.

### Kinetische Analyse der Plasminogen Aktivierung durch indirekten Chromogen-Test

Indirekte chromogene Tests der t-PA-Aktivität verwenden die Substrate Lys-Plasminogen (American Diagnostica) und Spectrozyme PL (American Diagnostica) und wurden gemäß Madison E.L., Goldsmith E.J., Gerard R.D., Gething M.-J., Sambrook J.F. (1989) Nature 339 721-724; Madison E.L., Goldsmith E.J., Gerard R.D., Gething M.J., Sambrook J.F. and Bassel-Duby R.S. (1990) Proc. Natl. Acad. Sci. U.S.A. 87, 3530-3533 sowie Madison E.L., Goldsmith E.J., Gething M.J., Sambrook J.F. und Gerard R.D. (1990) J. Biol. Chem. 265, 21423-21426 durchgeführt. Tests wurden sowohl in der An- als auch der Abwesenheit des Co-Faktors DESAFIB (American Diagnostica) durchgeführt. DESAFIB, eine Präparation löslicher Fibrin- Monomere, wurde durch die Spaltung hochreinen humanen Fibrinogens durch die Protease Batroxobin gewonnen. Batroxobin spaltet dabei die Arg¹⁶-Gly¹⁷-Bindung in der Aα-Kette des Fibrinogens und setzt dabei Fibrinopeptid A frei. Das resultierende des-AA-Fibrinogen in Form von Fibrin I Monomeren ist in Anwesenheit des Peptids Gly-Pro-Arg-Pro löslich. Die Konzentration des Lys-Plasminogens wurde in Anwesenheit von DESAFIB von 0,0125 bis zu 0,2 µM variiert, in Abwesenheit des Co-Faktors von 0,9 bis 16 µM.

### Indirekte Chromogen-Tests in Gegenwart verschiedener Stimulatoren

Indirekte chromogene Standardtests wurden gemäß der oben zitierten Veröffentlichungen durchgeführt. Dabei wurden Ansätze von 100 µl Gesamtvolumen mit 0,25-1 ng Enzym, 0,2 µM Lys-Plasminogen und 0,62 mM Spectrozym PL verwendet. Die Tests wurden in Anwesenheit von entweder Puffer, 25 µg/ml DESAFIB, 100 µg/ml Cyanogen Bromid Fragmenten von Fibrinogen (American Diagnostica) oder 100 µg/ml des stimulatorischen 13-Aminosäure-Peptids P368 durchgeführt. Die Analysen wurden in Mikrotiter-Platten durchgeführt und die optische Dichte bei 405 nm Wellenlänge 1 h lang alle 30 sec in einem "Molecular Devices Thermomax" gemessen. Die Reaktionstemperatur betrug 37 °C.

### Referenzen:

Baranes D, Lederfein D, Huang YY, Chen M, Bailey CH, Kandel ER. 1998. Tissue plasminogen activator contributes to the late phase of LTP and to synaptic growth in the hippocampal mossy fiber pathway. Neuron 21:813-25.
Bringmann P, Gruber D, Liese A, Toschi L, Kratzchmar J, Schleuning WD Donner P. 1995. Structural features mediating fibrin selectivity of vampire bat plasminogen activators. J Biol Chem 270:25596-25603.
Callaway JK, Knight MJ, Watkins DJ, Beart PM, Jarrott B, Delaney PM. 2000. A novel, rapid, computerized method for quantitation of neuronal damage in a rat model of stroke. J Neurosci Methods 102:53-60
Carmeliet P, Schoonjans L, Kieckens L, Ream B, Degen J, Bronson R, De Vos R, van den Oord JJ, Collen D, Mulligan RC. 1994. Physiological consequences of loss of plasminogen activator gene function in mice. Nature 368:419-424.
Cartwright T. 1974. The plasminogen activator of vampire bat saliva. Blood 43:317-326.
Chen ZL Strickland S. 1997. Neuronal death in the hippocampus is promoted by plasmin-catalysed degradation of laminin. Cell 91:917-925.
Chen, Z-L, Indyk, J.A., Bugge, T.H., Kombrinck, K.W., and Strickland S. 1999. Neuronal Death and blood-brain barrier breakdown after excitotoxic injury are independent processes. J. Neuroscience 19:9813-9820
Frey U, Muller M, Kuhl D. 1996. A different form of long-lasting potentiation revealed in tissue plasminogen activator mutant mice. J Neurosci 16:2057-2063.
Gardell SJ, Duong LT, Diehl RE, York JD, Hare TR, Register RB, Jacobs JW, Dixon RA, Friedman PA. 1989. Isolation, characterization, and cDNA cloning of a vampire bat salivary plasminogen activator. J Biol Chem 264:17947-17952.
Gardell SJ, Ramjit DR, Stabilito II, Fujita T, Lynch JJ, Cuca GC, Jain D, Wang SP, Tung JS, Mark GE, et al. 1991. Effective thrombolysis without marked plasminemia after bolus intravenous administration of vampire bat salivary plasminogen activator in rabbits. Circulation 84:244-253.
Granelli-Piperno, A and Reich, E. 1974. A study of proteases and protease inhibitor complexes in biological fluids. J. Exp. Med. 148: 223-234.
Huang YY, Bach ME, Lipp HP, Zhuo M, Wolfer DP, Hawkins RD, Schoonjans L, Kandel ER, Godfraind JM, Mulligan R, Collen D, Carmeliet P. 1996. Mice lacking the gene encoding tissue-type plasminogen activator show a selective interference with late-phase long-term potentiation in both Schaffer collateral and mossy fiber pathways. Proc Natl Acad Sci U S A. 93:8699-86704.
Kratzschmar J, Haendler B, Langer G, Boidol W, Bringmann P, Alagon A, Donner P, Schleuning WD. 1991. The plasminogen activator family from the salivary gland of the vampire bat Desmodus rotundus: cloning and expression. Gene 105:229-237.
Madani R, Hulo S, Toni N, Madani H, Steimer T, Muller D, Vassalli JD. (1999) Enhanced hippocampal long-term potentiation and learning by increased neuronal expression of tissue-type plasminogen activator in transgenic mice. EMBO J.18:3007-3012
Mellott MJ, Stabilito II, Holahan MA, Cuca GC, Wang S, Li P, Barrett JS, Lynch JJ, Gardell SJ. 1992. Vampire bat salivary plasminogen activator promotes rapid and sustained reperfusion concomitant systemic plasminogen activation in a canine model of arterial thrombosis. Arterioscler. Thromb. 12:212-221.
Muschick, P., Zeggert D., Donner, P., Witt, W. 1993. Thrombolytic properties of Desmodus (vampire bat) plasminogen activator DSPAα1, Alteplase and streptokinase following intravenous bolus injection in a rabbit model of carotid artery thrombolysis. Fibrinolysis 7: 284-290.
Müller, C.M., Griesinger, C.B. 1998. Tissue plasminogen activator mediates reverse occlusion plasticity in visual cortex. Nature Neuroscience. 1: 47-53.
National Institute of Neurological Disorders and stroke rt-PA study group. 1995. New. Engl. J. Med. 333: 1581-1587
Nicole, O., Docagne, F., Ali, C., Margaill, I., Carmeliet, P., MacKenzie, E.T. Vivien, D. & Buisson, A. (2001) The proteolytic activity of tissue-plasminogen activator enhances NMDA receptor-mediated signaling. Nat Med 7, 59-64.
Rogove AD, Siao C, Keyt B, Strickland S, Tsirka SE. 1999. Activation of microglia reveals a non-proteolytic cytokine function for tissue plasminogen activator in the central nervous system. J Cell Sci 112:4007-4016.
Schleuning WD, Alagon A, Boidol W, Bringmann P, Petri T, Kratzschmar J, Haendler B, Langer G, Baldus B, Witt W, et al. 1992. Plasminogen activators from the saliva of Desmodus rotundus (common vampire bat): unique fibrin specificity. Ann N Y Acad Sci 667:395-403.
Seeds, N.W., Williams, B.L., Bickford, P.C. 1995. Tissue plasminogen activator induction in purkinje neurons after cerebellar motor learning. Science. 270:1992-1994.
Stewart RJ, Fredenburgh JC, Weitz JI. 1998. Characterization of the interactions of plasminogen and tissue and vampire bat plasminogen activators with fibrinogen, fibrin, and the complex of D-dimer noncovalently linked to fragment E. J Biol Chem 273:18292-18299.
Traynelis, SF, Lipton, SA. 2001. Is tissue plasminogen activator a threat to neurons? Nature Medicine, 7:17-18.
Toschi L, Bringmann P, Petri T, Donner P, Schleuning WD. 1998. Fibrin selectivity of the isolated protease domains of tissue-type and vampire bat salivary gland plasminogen activators. Eur J Biochem 252:108-112.
Tsirka SE, Gualandris A, Amaral DG, Strickland S. 1995. Excitotoxin-induced neuronal degeneration and seizure are mediated by tissue plasminogen activator. Nature. 377:340-344.
Tsirka, S., Rogove, A. D., and Strickland, S. 1996. Neuronal cell death and tPA. Nature 384: 123-124
Tsirka SE, Rogove AD, Bugge TH, Degen JL, Strickland S. 1997. An extracellular proteolytic cascade promotes neuronal degeneration in the mouse hippocampus. J Neurosci 17:543-552
Tsirka SE, Bugge TH, Degen JL, Strickland S. 1997. Neuronal death in the central nervous system demonstrates a non-fibrin substrate for plasmin. Proc Natl Acad Sci USA 94:9779-9781.
Wang YF, Tsirka SE, Strickland S, Stieg PE, Soriano SG, Lipton SA. 1998. Tissue plasminogen activator (tPA) increases neuronal damage after focal cerebral ischemia in wild-type and tPA-deficient mice. Nat Med. 4:228-231
Walker JB, Nesheim ME. 2001. A kinetic analysis of the tissue plasminogen activator and DSPAalpha1 cofactor activities of untreated and TAFla treated soluble fibrin degradation products of varying size. J Biol Chem 276:3138-3148.
Witt W, Maass B, Baldus B, Hildebrand M, Donner P, Schleuning WD. 1994. Coronary thrombolysis with Desmodus salivary plasminogen activator in dogs. Fast and persistent recanalization by intravenous bolus administration. Circulation. 90:421-426.

## Patentansprüche

1. Verwendung einer Mutante eines Plasminogen Aktivierenden Faktors, dessen Fibrinspezifität im Vergleich zum Wildtyp t-PA erhöht ist, zur Herstellung eines Medikaments zur therapeutischen Behandlung des Schlaganfalls beim Menschen nach Ablauf von drei Stunden seit Beginn des Schlaganfalls, ohne die Kombination mit einem weiteren Therapeutikum.

2. Verwendung eines Plasminogen Aktivierenden Faktors nach Anspruch 1, **gekennzeichnet durch** die therapeutische Behandlung des Schlaganfalls beim Menschen nach Ablauf von sechs Stunden seit Beginn des Schlaganfalls.

3. Verwendung eines Plasminogen Aktivierenden Faktors nach einem der Ansprüche 1 oder 2, **gekennzeichnet durch** die therapeutische Behandlung des Schlaganfalls beim Menschen nach Ablauf von neun Stunden seit Beginn des Schlaganfalls.

4. Verwendung eines Plasminogen Aktivierenden Faktors nach einem der vorherigen Ansprüche, **dadurch gekennzeichnet, daß** der Plasminogen-Aktivierende Faktor mindestens einen Histidin- oder Serin-Rest umfaßt, der gemeinsam mit einem Aspartatrest mindestens einen Teil einer Zymogentriade bildet.

5. Verwendung eines Plasminogen Aktivierenden Faktors nach Anspruch 4, **dadurch gekennzeichnet, daß** der Plasminogen-Aktivierende Faktor ausgewählt ist aus der Gruppe folgender Mutanten des t-PA: t-PA/R275E; t-PA/R275E, F305H; t-PA/R275E, F305H, A292S.

6. Verwendung eines Plasminogen Aktivierenden Faktors nach einem der vorherigen Ansprüche, **dadurch gekennzeichnet, daß** der Plasminogen Aktivierende Faktor eine Punktmutation des Asp194 aufweist, die die Stabilisierung der katalytisch aktiven Konformation des Plasminogen Aktivierenden Faktors bei Abwesenheit von Fibrin vermindert.

7. Verwendung nach Anspruch 6, **dadurch gekennzeichnet, daß** Asp194 durch Glutamat oder Asparagin substituiert ist.

8. Verwendung eines Plasminogen Aktivierenden Faktors nach Anspruch 1, **dadurch gekennzeichnet, daß** der Plasminogen Aktivierende Faktor wenigstens eine Mutation in seiner Autolyse-Schleife aufweist, die die Ausbildung von funktionellen Wechselwirkungen zwischen Plasminogen und Plasminogen Aktivierendem Faktor bei Abwesenheit von Fibrin vermindert.

9. Verwendung nach Anspruch 8, **gekennzeichnet durch** mindestens eine Mutation in der Autolyse-Schleife in den Aminosäurepositionen 420 bis 423 des Wildtyp t-PA oder in dazu homologen Positionen.

10. Verwendung nach Anspruch 9, **gekennzeichnet durch** eine Mutation ausgewählt aus der Gruppe folgender Mutanten: L420A, L420E, S421 G, S421 E, P422A, P422G, P422E, F423A und F423E.

11. Verwendung eines Plasminogen Aktivierenden Faktors nach einem der vorherigen Ansprüche, **dadurch gekennzeichnet, daß** der Plasminogen Aktivierende Faktor als Zymogen mindestens eine Punktmutation aufweist, die die Katalyse durch Plasmin verhindert.

12. Verwendung eines Plasminogen Aktivierenden Faktors nach Anspruch 11, **dadurch gekennzeichnet, daß** die Punktmutation an den Positionen 15 oder 275 des t-PA oder dazu homologer Positionen lokalisiert ist.

13. Verwendung nach Anspruch 12, **gekennzeichnet durch** Glutamat in der Position 15 oder 275.

14. Verwendung nach Anspruch 1, **dadurch gekennzeichnet, dass** der Plasminogen-Aktivierende Faktor eine Punktmutation in Position 194 aufweist, die die Stabilisierung der katalytisch aktiven Konformation des Plasminogen-Aktivierenden Faktors bei Abwesenheit von Fibrin vermindert.

15. Verwendung nach Anspruch 14, **gekennzeichnet durch** ein Phe194.

16. Verwendung nach Anspruch 1, **gekennzeichnet durch** einen Plasminogen-Aktivierenden Faktor mit einer Aminosäuresequenz mit SEQ. ID Nr. 1.

17. Verwendung nach Anspruch 1, **gekennzeichnet durch** einen Plasminogen Aktivierenden Faktor mit einer Aminosäuresequenz gemäß SEQ. ID Nr. 2.

## Claims

1. Use of a mutant of a plasminogen activating factor, the fibrin specificity of which is elevated in comparison to the wild type t-PA, for producing a medicament for the therapeutic treatment of stroke in humans three hours after stroke onset, without the combination with a further therapeutic agent.

2. Use of a plasminogen activating factor according to claim 1, **characterised by** the therapeutic treatment of a stroke in humans after six hours have passed since the onset of the stroke.

3. Use of a plasminogen activating factor according to either of claims 1 or 2, **characterised by** the therapeutic treatment of a stroke in humans after nine hours have passed since the onset of the stroke.

4. Use of a plasminogen activating factor according to any one of the preceding claims, **characterised in that** the plasminogen activating factor comprises at least a histidine or serine residue, which together with an aspartate residue forms at least a part of a cymogene triade.

5. Use of a plasminogen activating factor according to claim 4, **characterised in that** the plasminogen activating factor is selected from the group of following t-PA mutants: t-PA/R275E; t-PA/R275E, F305H; t-PA/R275E, F305H, A292S.

6. Use of a plasminogen activating factor according to any one of the preceding claims, **characterised in that** the plasminogen activating factor has a point mutation of the Asp194, which reduces the stabilisation of the catalytically active conformation of the plasminogen activating factor in the absence of fibrin.

7. Use according to claim 6, **characterised in that** Asp194 is substituted by glutamate or asparagine.

8. Use of a plasminogen activating factor according to claim 1, **characterised in that** the plasminogen activating factor has at least one mutation in its autolysis loop, which reduces the formation of functional interactions between plasminogen and plasminogen activating factor in the absence of fibrin.

9. Use according to claim 8, **characterised by** at least one mutation in the autolysis loop in the amino acid positions 420 to 423 of the wild type t-PA or in positions which are homologous thereto.

10. Use according to claim 9, **characterised by** a mutation selected from the group of following mutants: L420A, L420E, S421 G, S421 E, P422A, P422G, P422E, F423A and F423E.

11. Use of a plasminogen activating factor according to any one of the preceding claims, **characterised in that** the plasminogen activating factor as a cymogene has at least one point mutation preventing the catalysis by plasmin.

12. Use of a plasminogen activating factor according to claim 11, **characterised in that** the point mutation is located at the positions 15 or 275 of t-PA or positions which are homologous thereto.

13. Use according to claim 12, **characterised by** glutamate in the position 15 or 275.

14. Use according to claim 1, **characterised in that** the plasminogen activating factor has a point mutation in position 194, which reduces the stabilisation of the catalytically active conformation of the plasminogen activating factor in the absence of fibrin.

15. Use according to claim 14, **characterised by** a Phe194.

16. Use according to claim 1, **characterised by** a plasminogen activating factor with an amino acid sequence with SEQ. ID No. 1.

17. Use according to claim 1, **characterised by** a plasminogen activating factor with an amino acid sequence in accordance with SEQ. ID No. 2.

## Revendications

1. Utilisation d'un mutant d'un facteur activateur du plasminogène, dont la spécificité à la fibrine est augmentée par rapport au type sauvage t-PA, pour fabriquer un médicament destiné au traitement thérapeutique de l'accident vasculaire cérébral chez l'homme après un écoulement d'une durée de trois heures depuis le début de l'accident vasculaire cérébral, sans combinaison avec un autre agent thérapeutique.

2. Utilisation d'un facteur activateur du plasminogène selon la revendication 1, **caractérisée par** le traitement thérapeutique de l'accident vasculaire cérébral chez l'homme après un écoulement d'une durée de six heures depuis le début de l'accident vasculaire cérébral.

3. Utilisation d'un facteur activateur du plasminogène selon l'une des revendications 1 ou 2, **caractérisée par** le traitement thérapeutique de l'accident vasculaire cérébral chez l'homme après un écoulement d'une durée de neuf heures depuis le début de l'accident vasculaire cérébral.

4. Utilisation d'un facteur activateur du plasminogène selon l'une des revendications précédentes, **caractérisée en ce que** le facteur activateur du plasminogène comporte au moins un résidu histidine ou sérine qui forme conjointement à un résidu aspartate au moins une partie d'une triade zymogène.

5. Utilisation d'un facteur activateur du plasminogène selon la revendication 4, **caractérisée en ce que** le facteur activateur du plasminogène est sélectionné parmi le groupe composé des mutants suivants du t-PA : t-PA/R275E ; t-PA/R275E, F305H ; t-PA/R275E, F305H, A292S.

6. Utilisation d'un facteur activateur du plasminogène selon l'une des revendications précédentes, **caractérisée en ce que** le facteur activateur du plasminogène présente une mutation ponctuelle de Asp194 qui réduit la stabilisation de la conformation catalytiquement active du facteur activateur du plasminogène en absence de fibrine.

7. Utilisation selon la revendication 6, **caractérisée en ce que** Asp194 est substituée par le glutamate ou l'asparagine.

8. Utilisation d'un facteur activateur du plasminogène selon la revendication 1, **caractérisée en ce que** le facteur activateur du plasminogène présente au moins une mutation dans sa boucle d'autolyse qui réduit la formation d'interactions fonctionnelles entre le plasminogène et le facteur activateur du plasminogène en absence de fibrine.

9. Utilisation selon la revendication 8, **caractérisée par** au moins une mutation dans la boucle d'autolyse, dans les positions d'acides aminés 420 à 423 du type sauvage t-PA ou dans des positions qui leur sont homologues.

10. Utilisation selon la revendication 9, **caractérisée par** une mutation sélectionnée parmi le groupe des mutants suivants : L420A, L420E, S421 G, S421 E, P422A, P422G, P422E, F423A et F423E.

11. Utilisation d'un facteur activateur du plasminogène selon l'une des revendications précédentes, **caractérisée en ce que** le facteur activateur du plasminogène en tant que zymogène présente au moins une mutation ponctuelle qui empêche la catalyse par la plasmine.

12. Utilisation d'un facteur activateur du plasminogène selon la revendication 11, **caractérisée en ce que** la mutation ponctuelle est localisée au niveau des positions 15 ou 275 de t-PA ou de positions qui leur sont homologues.

13. Utilisation selon la revendication 12, **caractérisée par** du glutamate en position 15 ou 275.

14. Utilisation selon la revendication 1, **caractérisée en ce que** le facteur activateur du plasminogène présente une mutation ponctuelle en position 194 qui réduit la stabilisation de la conformation catalytiquement active du facteur activateur du plasminogène en absence de fibrine.

15. Utilisation selon la revendication 14, **caractérisée par** une Phe 194.

16. Utilisation selon la revendication 1, **caractérisée par** un facteur activateur du plasminogène ayant une séquence d'acides aminés d'ID SEQ. n° 1.

17. Utilisation selon la revendication 1, **caractérisée par** un facteur activateur du plasminogène ayant une séquence d'acides aminés d'ID SEQ. n° 2.
